(19) **Europäisches Patentamt / European Patent Office / Office européen des brevets**

(11) **EP 4 393 940 A1**

(12) **EUROPEAN PATENT APPLICATION**
published in accordance with Art. 153(4) EPC

(43) Date of publication:
**03.07.2024 Bulletin 2024/27**

(21) Application number: **22860475.7**

(22) Date of filing: **23.08.2022**

(51) International Patent Classification (IPC):
*C07K 14/475* (2006.01)    *A61K 38/18* (2006.01)
*A61K 47/68* (2017.01)    *A61P 3/04* (2006.01)

(52) Cooperative Patent Classification (CPC):
**A61P 3/00; A61K 38/18; A61P 3/04; A61P 3/06;**
**A61P 3/08; C07K 14/475; C07K 16/46;**
C07K 2317/52; C07K 2317/94; C07K 2319/30

(86) International application number:
**PCT/CN2022/114143**

(87) International publication number:
**WO 2023/025129 (02.03.2023 Gazette 2023/09)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **24.08.2021 CN 202110977378**

(71) Applicant: **Sunshine Lake Pharma Co., Ltd.**
**Dongguan, Guangdong 523000 (CN)**

(72) Inventors:
• **YAN, Jiangyu**
**Dongguan, Guangdong 523871 (CN)**
• **CAO, Rongbo**
**Dongguan, Guangdong 523871 (CN)**
• **XIAO, Lin**
**Dongguan, Guangdong 523871 (CN)**
• **ZENG, Junnan**
**Dongguan, Guangdong 523871 (CN)**
• **HUANG, Limei**
**Dongguan, Guangdong 523871 (CN)**

• **WEI, Xiling**
**Dongguan, Guangdong 523871 (CN)**
• **LI, Zhaofeng**
**Dongguan, Guangdong 523871 (CN)**
• **GONG, Qingwei**
**Dongguan, Guangdong 523871 (CN)**
• **YAN, Xingguo**
**Dongguan, Guangdong 523871 (CN)**
• **LI, Jing**
**Dongguan, Guangdong 523871 (CN)**
• **HUANG, Danxia**
**Dongguan, Guangdong 523871 (CN)**
• **CHEN, Xiaofeng**
**Dongguan, Guangdong 523871 (CN)**
• **LI, Wenjia**
**Dongguan, Guangdong 523871 (CN)**

(74) Representative: **Hoffmann Eitle**
**Patent- und Rechtsanwälte PartmbB**
**Arabellastraße 30**
**81925 München (DE)**

Remarks:
The complete document including Reference Table(s) and the Sequence Listing(s) can be downloaded from the EPO website

(54) **GDF15 FUSION PROTEIN AND USE THEREOF**

(57)    A GDF15 fusion protein and a method for treating obesity using same. The fusion protein comprises an Fc variant and a GDF15 active domain, wherein the Fc variant has an amino acid substitution at position 356 and/or position 439 of the IgG Fc according to EU numbering and still has the ability to form a homodimer. By means of fusing the Fc variant with the GDF15 active domain, the Fc-GDF15 fusion protein has significantly improved physical and chemical properties and recombinant expression level, has an in vitro activity comparable to or better than that of a natural GDF15 molecule, and has a significantly prolonged in vivo cyclic half-life, which may support the dosing frequency once every two weeks or even once a month.

**(Cont. next page)**

Figure 4

## Description

This application claims the priority and benefit of Chinese Patent Application No. 202110977378.7, filed with the State Intellectual Property Office of China on August 24, 2021, which is incorporated herein by reference in its entirety.

## FIELD OF THE INVENTION

The invention relates to the technical field of biomedicine, in particular to GDF15 fusion proteins and a method for treating obesity using same.

## BACKGROUND ART

Obesity refers to a chronic metabolic disease that occurs due to the increase in the volume and number of fat cells in the body and the abnormal distribution of adipose tissue, which is caused by the interaction of multiple factors, and at least 2.8 million deaths per year can be attributed to overweight or obesity. The main policy-making bodies of American Medical Association (AMA) makes it clear that obesity should be considered not just a risk factor for disease, but a real disease, the American Association of Clinical Endocrinologists (AACE) states that being overweight or obese increases the risk of breast cancer, coronary heart disease, type II diabetes, gallbladder disease, osteoarthritis, colon cancer, hypertension and stroke.

Obesity management includes lifestyle changes, medications, and bariatric surgery. Lifestyle changes require a team of professionals to develop and maintain a protocol, but are often associated with high rates of recurrence, and bariatric surgery is associated with a risk of death and surgical complications, and it is costly, and it is commonly used for severe obesity. Given the current limitations of lifestyle changes and bariatric surgery, the use of medications to treat obesity is essential. Authoritative US guidelines recommend treatment with anti-obesity drugs when a body mass index $\geq 30$ kg/m$^2$ or a body mass index $\geq 27$ kg/m$^2$ with at least one weight-related complication.

Growth differentiation factor 15 (GDF15), also known as macrophage inhibitory cytokine-1 (MIC-1), placental transforming growth factor-□ (PTGF-□), placental bone morphogenetic factor (PLAB), prostate-derived factor (PDF), non-steroidal anti-inflammatory drug-activated gene (NAG-1), is a distant member of the transforming growth factor (TGF-□) superfamily. The mature GDF15 polypeptide contains 112 amino acids, while the biologically active GDF15 circulating *in vivo* is a homodimeric protein (24.5kD) formed by two polypeptides through interchain disulfide bonds.

It has been reported that elevated circulating levels of GDF15 is associated with reduced food intake and weight loss in patients with advanced cancer (Johnen H et al, Nat Med, 2007). Furthermore, both transgenic mice with high expression of GDF15 and mice administered recombinant GDF15 exhibited reduced body weight and food intake, and had improved glucose tolerance (Xiong Y et al., Sci Transl Med, 2017), which indicating that GDF15 has the potential to treat diseases such as obesity, type II diabetes (T2D), and nonalcoholic steatohepatitis (NASH).

The circulating half-life (t1/2) of native GDF15 dimer *in vivo* is short, only about 2-3 hours, which greatly limits its clinical therapeutic application. Conventional methods to prolong the circulating half-life of biopharmaceuticals include fusing the pharmaceutical molecule with the Fc fragment of antibody. However, due to the characteristics of the three-dimensional structure of GDF15 dimer and Fc fragment dimer molecule, direct fusion of GDF15 and Fc fragment will greatly affect the overall stability of the fusion molecule and the yield of recombinant expression.

Therefore, it's very necessary to develop stable therapeutic GDF15 fusion protein molecules with longer half-life and simple process for the treatment of obesity.

## SUMMARY

The purpose of the present invention is to provide a method for treating obesity-related diseases using Fc-GDF15 fusion protein. The GDF15 fusion protein of the present invention has the advantages of optimal physicochemical properties, long *in vivo* half-life and duration of efficacy, simple manufacturing process, *etc.,* and can be used for the treatment of metabolic-related diseases, such as obesity, type II diabetes, NASH, dyslipidemia, *etc.,* especially obesity.

In the first aspect, the invention provides a method for treating obesity-related diseases using Fc-GDF15 fusion protein.

To this end, the present invention provides a fusion protein, in some embodiments, the fusion protein comprises a GDF15 active domain and an Fc variant; the C-terminal of the Fc variant is linked directly or via a peptide linker to the N-terminal of the GDF15 active domain;

the Fc variant comprises amino acid substitutions at position 356 and/or position 439 of the IgG Fc according to EU numbering.

In some embodiments, the Fc variant retains the ability to form homodimers.

In some embodiments, the IgG Fc is selected from one of IgG1 Fc, IgG2 Fc, IgG3 Fc and IgG4 Fc.

In some embodiments, the IgG Fc is human IgG1 Fc. In some embodiments, the IgG Fc is human IgG4 Fc.

Further, the Fc variant comprises an amino acid substitution at position 356 of IgG Fc with amino acids other than D, E and C according to EU numbering. For example, the amino acid at position 356 of IgG Fc is substituted with one of the following group: G, S, A, T, V, N, L, I, Q, Y, F, H, P, M, K, R. Further, the Fc variant comprises an amino acid substitution at position 439 of IgG Fc with amino acids other than R, H, K and C according to EU numbering. For example, the amino acid at position 439 of IgG Fc is substituted with one of the following group: G, S, A, T, V, D, N, L, I, E, Q, Y, F, P, M.

In some embodiments, the Fc variant comprises one of the following mutations in IgG Fc according to EU numbering: E356G, E356S, E356A, E356T, E356V, E356N, E356L, E356I, E356Q, E356Y, E356F, E356H, E356P, E356M, E356K, E356R, and/or one of K439G, K439S, K439A, K439T, K439V, K439D, K439N, K439L, K439I, K439E, K439Q, K439Y, K439F, K439H, K439P, K439M.

In preferred embodiments, the Fc variant comprises the following mutations in IgG Fc according to EU numbering: one of E356R, E356Q, E356A, E356N, and/or one of K439D, K439E, K439Q, K439A, K439N.

In some embodiments, the Fc variant comprises one of the following mutations in IgG Fc according to EU numbering: K439D, K439E, K439Q, K439A, K439N. In preferred embodiments, the Fc variant comprises one of the following mutations in IgG Fc according to EU numbering: K439D, K439E, K439Q.

In some embodiments, the Fc variant comprises one of the following mutations in IgG Fc according to EU numbering: E356R, E356Q, E356A, E356N. In preferred embodiments, the Fc variant comprises the following mutation in IgG Fc according to EU numbering: E356R.

In some embodiments, the Fc variant further comprises the following mutations: amino acid substitutions at position 234 and position 235 of the IgG Fc according to EU numbering to AA, and/or amino acid deletion at position 447. For example, for IgG1 Fc, the following mutations are also included: L234A and L235A, and/or amino acid deletion at position 447; for IgG4 Fc, the following mutations are also included: F234A and L235A, and/or amino acid deletion at position 447.

In a certain embodiment, the Fc variant has only one of the following amino acid substitutions in the IgG Fc according to EU numbering:

amino acid substitution at position 356 according to EU numbering;
amino acid substitution at position 439 according to EU numbering;
amino acid substitutions at position 356 and position 439 according to EU numbering;
amino acid substitutions at position 356, position 234 and position 235 according to EU numbering;
amino acid substitutions at position 439, position 234 and position 235 according to EU numbering;
amino acid substitutions at position 356, position 439, position 234 and position 235 according to EU numbering;
the amino acid substitutions described at the respective positions have the meaning described in the present invention.

In a certain embodiment, the amino acid sequence of the Fc variant comprises one of the following sequences: SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 27, SEQ ID NO: 29, SEQ ID NO: 30, SEQ ID NO:31, SEQ ID NO:32, SEQ ID NO:33, SEQ ID NO:34, SEQ ID NO:35, SEQ ID NO:36, SEQ ID NO:37, SEQ ID NO:38, SEQ ID NO:39, SEQ ID NO:40, SEQ ID NO:41, SEQ ID NO:42, SEQ ID NO:43, SEQ ID NO:44, SEQ ID NO:45.

Table 1 Fc variants

| SEQ ID NO: | Amino Acid Sequence | Note |
|---|---|---|
| 1 | GPSVFLFPPKPKDTLMISRTPEVTCVVVDVSQEDPEVQFNWY VDGVEVHNAKTKPREEQFNSTYRVVSVLTVLHQDWLNGKEY KCKVSNKGLPSSIEKTISKAKGQPREPQVYTLPPSQEEMTKNQ VSLTCLVKGFYPSDIAVEWESNGQPENNYKTTPPVLDSDGSFF LYSRLTVDKSRWQEGNVFSCSVMHEALHNHYTQ**E**SLSLSLG | Fc (G, K439E) |

(continued)

| SEQ ID NO: | Amino Acid Sequence | Note |
|---|---|---|
| 2 | GGPSVFLFPPKPKDTLMISRTPEVTCVVVDVSQEDPEVQFNW YVDGVEVHNAKTKPREEQFNSTYRVVSVLTVLHQDWLNGKE YKCKVSNKGLPSSIEKTISKAKGQPREPQVYTLPPSQEEMTKN QVSLTCLVKGFYPSDIAVEWESNGQPENNYKTTPPVLDSDGS FFLYSRLTVDKSRWQEGNVFSCSVMHEALHNHYTQ**E**SLSLSL G | Fc (GG, K439E) |
| 3 | E**AA**GGPSVFLFPPKPKDTLMISRTPEVTCVVVDVSQEDPEVQF N**W**YVDGVEVHNAKTKPREEQFNSTYRVVSVLTVLHQDWLN GKEYKCKVSNKGLPSSIEKTISKAKGQPREPQVYTLPPSQEEM TKNQVSLTCLVKGFYPSDIAVEWESNGQPENNYKTTPPVLDS DGSFFLYSRLTVDKSRWQEGNVFSCSVMHEALHNHYTQ**E**SLS LSLG | Fc (AA, K439E) |
| 4 | APE**AA**GGPSVFLFPPKPKDTLMISRTPEVTCVVVDVSQEDPEV QFN**W**YVDGVEVHNAKTKPREEQFNSTYRVVSVLTVLHQDW LNGKEYKCKVSNKGLPSSIEKTISKAKGQPREPQVYTLPPSQE EMTKNQVSLTCLVKGFYPSDIAVEWESNGQPENNYKTTPPVL DSDGSFFLYSRLTVDKSRWQEGNVFSCSVMHEALHNHYTQ**E** SLSLSLG | Fc (APEAA , K439E) |
| 5 | GPSVFLFPPKPKDTLMISRTPEVTCVVVDVSQEDPEVQFNWY VDGVEVHNAKTKPREEQFNSTYRVVSVLTVLHQDWLNGKEY KCKVSNKGLPSSIEKTISKAKGQPREPQVYTLPPSQEEMTKNQ VSLTCLVKGFYPSDIAVEWESNGQPENNYKTTPPVLDSDGSFF LYSRLTVDKSRWQEGNVFSCSVMHEALHNHYTQ**D**SLSLSLG | Fc (G, K439D) |
| 6 | GGPSVFLFPPKPKDTLMISRTPEVTCVVVDVSQEDPEVQFNW YVDGVEVHNAKTKPREEQFNSTYRVVSVLTVLHQDWLNGKE YKCKVSNKGLPSSIEKTISKAKGQPREPQVYTLPPSQEEMTKN QVSLTCLVKGFYPSDIAVEWESNGQPENNYKTTPPVLDSDGS FFLYSRLTVDKSRWQEGNVFSCSVMHEALHNHYTQ**D**SLSLSL G | Fc (GG, K439D) |
| 7 | E**AA**GGPSVFLFPPKPKDTLMISRTPEVTCVVVDVSQEDPEVQF N**W**YVDGVEVHNAKTKPREEQFNSTYRVVSVLTVLHQDWLN GKEYKCKVSNKGLPSSIEKTISKAKGQPREPQVYTLPPSQEEM TKNQVSLTCLVKGFYPSDIAVEWESNGQPENNYKTTPPVLDS DGSFFLYSRLTVDKSRWQEGNVFSCSVMHEALHNHYTQ**D**SL SLSLG | Fc (AA, K439D) |
| 8 | APE**AA**GGPSVFLFPPKPKDTLMISRTPEVTCVVVDVSQEDPEV QFN**W**YVDGVEVHNAKTKPREEQFNSTYRVVSVLTVLHQDW LNGKEYKCKVSNKGLPSSIEKTISKAKGQPREPQVYTLPPSQE EMTKNQVSLTCLVKGFYPSDIAVEWESNGQPENNYKTTPPVL DSDGSFFLYSRLTVDKSRWQEGNVFSCSVMHEALHNHYTQ**D** SLSLSLG | Fc (APEAA K439D) |
| 9 | GPSVFLFPPKPKDTLMISRTPEVTCVVVDVSQEDPEVQFNWY VDGVEVHNAKTKPREEQFNSTYRVVSVLTVLHQDWLNGKEY KCKVSNKGLPSSIEKTISKAKGQPREPQVYTLPPSQ**K**EMTKNQ VSLTCLVKGFYPSDIAVEWESNGQPENNYKTTPPVLDSDGSFF LYSRLTVDKSRWQEGNVFSCSVMHEALHNHYTQKSLSLSLG | Fc (G, E356K) |
| 10 | GGPSVFLFPPKPKDTLMISRTPEVTCVVVDVSQEDPEVQFNW YVDGVEVHNAKTKPREEQFNSTYRVVSVLTVLHQDWLNGKE YKCKVSNKGLPSSIEKTISKAKGQPREPQVYTLPPSQ**K**EMTKN QVSLTCLVKGFYPSDIAVEWESNGQPENNYKTTPPVLDSDGS FFLYSRLTVDKSRWQEGNVFSCSVMHEALHNHYTQKSLSLSL G | Fc (GG, E356K) |

(continued)

| SEQ ID NO: | Amino Acid Sequence | Note |
|---|---|---|
| 11 | E**AA**GGPSVFLFPPKPKDTLMISRTPEVTCVVVDVSQEDPEVQF NWYVDGVEVHNAKTKPREEQFNSTYRVVSVLTVLHQDWLN GKEYKCKVSNKGLPSSIEKTISKAKGQPREPQVYTLPPSQ**K**EM TKNQVSLTCLVKGFYPSDIAVEWESNGQPENNYKTTPPVLDS DGSFFLYSRLTVDKSRWQEGNVFSCSVMHEALHNHYTQKSL SLSLG | Fc (AA, E356K) |
| 12 | APE**AA**GGPSVFLFPPKPKDTLMISRTPEVTCVVVDVSQEDPEV QFNWYVDGVEVHNAKTKPREEQFNSTYRVVSVLTVLHQDW LNGKEYKCKVSNKGLPSSIEKTISKAKGQPREPQVYTLPPSQ**K** EMTKNQVSLTCLVKGFYPSDIAVEWESNGQPENNYKTTPPVL DSDGSFFLYSRLTVDKSRWQEGNVFSCSVMHEALHNHYTQK SLSLSLG | Fc (APEAA , E356K) |
| 13 | GPSVFLFPPKPKDTLMISRTPEVTCVVVDVSQEDPEVQFNWY VDGVEVHNAKTKPREEQFNSTYRVVSVLTVLHQDWLNGKEY KCKVSNKGLPSSIEKTISKAKGQPREPQVYTLPPSQ**R**EMTKNQ VSLTCLVKGFYPSDIAVEWESNGQPENNYKTTPPVLDSDGSFF LYSRLTVDKSRWQEGNVFSCSVMHEALHNHYTQKSLSLSLG | Fc (G, E356R) |
| 14 | GGPSVFLFPPKPKDTLMISRTPEVTCVVVDVSQEDPEVQFNW YVDGVEVHNAKTKPREEQFNSTYRVVSVLTVLHQDWLNGKE YKCKVSNKGLPSSIEKTISKAKGQPREPQVYTLPPSQ**R**EMTKN QVSLTCLVKGFYPSDIAVEWESNGQPENNYKTTPPVLDSDGS FFLYSRLTVDKSRWQEGNVFSCSVMHEALHNHYTQKSLSLSL G | Fc (GG, E356R) |
| 15 | E**AA**GGPSVFLFPPKPKDTLMISRTPEVTCVVVDVSQEDPEVQF NWYVDGVEVHNAKTKPREEQFNSTYRVVSVLTVLHQDWLN GKEYKCKVSNKGLPSSIEKTISKAKGQPREPQVYTLPPSQ**R**EM TKNQVSLTCLVKGFYPSDIAVEWESNGQPENNYKTTPPVLDS DGSFFLYSRLTVDKSRWQEGNVFSCSVMHEALHNHYTQKSL SLSLG | Fc (AA, E356R) |
| 16 | APE**AA**GGPSVFLFPPKPKDTLMISRTPEVTCVVVDVSQEDPEV QFNWYVDGVEVHNAKTKPREEQFNSTYRVVSVLTVLHQDW LNGKEYKCKVSNKGLPSSIEKTISKAKGQPREPQVYTLPPSQ**R** EMTKNQVSLTCLVKGFYPSDIAVEWESNGQPENNYKTTPPVL DSDGSFFLYSRLTVDKSRWQEGNVFSCSVMHEALHNHYTQK SLSLSLG | Fc (APEAA , E356R) |
| 17 | APE**AA**GGPSVFLFPPKPKDTLMISRTPEVTCVVVDVSQEDPEV QFNWYVDGVEVHNAKTKPREEQFNSTYRVVSVLTVLHQDW LNGKEYKCKVSNKGLPSSIEKTISKAKGQPREPQVYTLPPSQE EMTKNQVSLTCLVKGFYPSDIAVEWESNGQPENNYKTTPPVL DSDGSFFLYSRLTVDKSRWQEGNVFSCSVMHEALHNHYTQ**Q** SLSLSLG | Fc (APEAA K439Q) |
| 18 | APE**AA**GGPSVFLFPPKPKDTLMISRTPEVTCVVVDVSQEDPEV QFNWYVDGVEVHNAKTKPREEQFNSTYRVVSVLTVLHQDW LNGKEYKCKVSNKGLPSSIEKTISKAKGQPREPQVYTLPPSQE EMTKNQVSLTCLVKGFYPSDIAVEWESNGQPENNYKTTPPVL DSDGSFFLYSRLTVDKSRWQEGNVFSCSVMHEALHNHYTQ**N** SLSLSLG | Fc (APEAA K439N) |
| 19 | APE**AA**GGPSVFLFPPKPKDTLMISRTPEVTCVVVDVSQEDPEV QFNWYVDGVEVHNAKTKPREEQFNSTYRVVSVLTVLHQDW LNGKEYKCKVSNKGLPSSIEKTISKAKGQPREPQVYTLPPSQE EMTKNQVSLTCLVKGFYPSDIAVEWESNGQPENNYKTTPPVL DSDGSFFLYSRLTVDKSRWQEGNVFSCSVMHEALHNHYTQ**A** SLSLSLG | Fc (APEAA K439A) |

(continued)

| SEQ ID NO: | Amino Acid Sequence | Note |
|---|---|---|
| 20 | APE**AA**GGPSVFLFPPKPKDTLMISRTPEVTCVVVDVSQEDPEV QFNW̲YVDGVEVHNAKTKPREEQFNSTYRVVSVLTVLHQDW LNGKEYKCKVSNKGLPSSIEKTISKAKGQPREPQVYTLPPSQ**Q** EMTKNQVSLTCLVKGFYPSDIAVEWESNGQPENNYKTTPPV̲L DSDGSFFLYSRLTVDKSRWQEGNVFSCSVMHEALHNHYTQK SLSLSLG | Fc (APEAA , E356Q) |
| 21 | APE**AA**GGPSVFLFPPKPKDTLMISRTPEVTCVVVDVSQEDPEV QFNW̲YVDGVEVHNAKTKPREEQFNSTYRVVSVLTVLHQDW LNGKEYKCKVSNKGLPSSIEKTISKAKGQPREPQVYTLPPSQ**N** EMTKNQVSLTCLVKGFYPSDIAVEWESNGQPENNYKTTPPV̲L DSDGSFFLYSRLTVDKSRWQEGNVFSCSVMHEALHNHYTQK SLSLSLG | Fc (APEAA , E356N) |
| 22 | APE**AA**GGPSVFLFPPKPKDTLMISRTPEVTCVVVDVSQEDPEV QFNW̲YVDGVEVHNAKTKPREEQFNSTYRVVSVLTVLHQDW LNGKEYKCKVSNKGLPSSIEKTISKAKGQPREPQVYTLPPSQ**A** EMTKNQVSLTCLVKGFYPSDIAVEWESNGQPENNYKTTPPV̲L DSDGSFFLYSRLTVDKSRWQEGNVFSCSVMHEALHNHYTQK SLSLSLG | Fc (APEAA , E356A) |
| 23 | E**AA**GGPSVFLFPPKPKDTLMISRTPEVTCVVVDVSQEDPEVQF NW̲YVDGVEVHNAKTKPREEQFNSTYRVVSVLTVLHQDWLN GKEYKCKVSNKGLPSSIEKTISKAKGQPREPQVYTLPPSQEEM TKNQVSLTCLVKGFYPSDIAVEWESNGQPENNYKTTPPVLDS DGSFFLYSRLTVDKSRWQEGNVFSCSVMHEALHNHYTQKSL SLSLG | Fc (AA) |
| 24 | E**AA**GGPSVFLFPPKPKDTLMISRTPEVTCVVVDVSQEDPEVQF NW̲YVDGVEVHNAKTKPREEQFNSTYRVVSVLTVLHQDWLN GKEYKCKVSNKGLPSSIEKTISKAKGQPREPQVYTLPPSQEEM TKNQVSLTCLVKGFYPSDIAVEWESNGQPENNYKTTPPVLDS DGSF**Q**L**E**SRLTVDKSRWQEGNVFSCSVMHEALHNHYTQKSL SLSLG | Fc (AA, F405Q/ Y407E) |
| 25 | APE**AA**GGPSVFLFPPKPKDTLMISRTPEVTCVVVDVSQEDPEV QFNW̲YVDGVEVHNAKTKPREEQFNSTYRVVSVLTVLHQDW LNGKEYKCKVSNKGLPSSIEKTISKAKGQPREPQVYTLPPSQE EMTKNQVSLTCLVKGFYPSDIAVEWESNGQPENNYKTTPPVL DSDGSFFLYSRLTVDKSRWQEGNVFSCSVMHEALHNHYTQK SLSLSLG | Fc (APEAA ) |
| 26 | GGPSVFLFPPKPKDTLMISRTPEVTCVVVDVSQEDPEVQFNW YVDGVEVHNAKTKPREEQFNSTYRVVSVLTVLHQDWLNGKE YKCKVSNKGLPSSIEKTISKAKGQPREPQVYTLPPSQEEMTKN QVSLTCLVKGFYPSDIAVEWESNGQPENNYKTTPPVLDSDGS FFLYSRLTVDKSRWQEGNVFSCSVMHEALHNHYTQKSLSLSL G | Fc (GG) |
| 27 | AESKYGPPCPPCPAPE**AA**GGPSVFLFPPKPKDTLMISRTPEVTC VVVDVSQEDPEVQFNW̲YVDGVEVHNAKTKPREEQFNSTYRV VSVLTVLHQDWLNGKEYKCKVSNKGLPSSIEKTISKAKGQPR EPQVYTLPPSQEEMTKNQVSLTCLVKGFYPSDIAVEWESNGQ PENNYKTTPPVLDSDGSFFLYSRLTVDKSRWQEGNVFSCSVM HEALHNHYTQ**E**SLSLSLG | Fc (Hinge, K439E) |
| 28 | GGPSVFLFPPKPKDTLMISRTPEVTCVVVDVSQEDPEVQFNW YVDGVEVHNAKTKPREEQFNSTYRVVSVLTVLHQDWLNGKE YKCKVSNKGLPSSIEKTISKAKGQPREPQVYTLPPSQEEMTKN QVSLTCLVKGFYPSDIAVEWESNGQPENNYKTTPPVLDSDGS F**Q**L**E**SRLTVDKSRWQEGNVFSCSVMHEALHNHYTQ**E**SLSLSL G | Fc (GG,F40 5Q/Y40 7E, K439E) |

(continued)

| SEQ ID NO: | Amino Acid Sequence | Note |
|---|---|---|
| 29 | GPSVFLFPPKPKDTLMISRTPEVTCVVVDVSHEDPEVKFNWY VDGVEVHNAKTKPREEQYNSTYRVVSVLTVLHQDWLNGKE YKCKVSNKALPAPIEKTISKAKGQPREPQVYTLPPSREEMTKN QVSLTCLVKGFYPSDIAVEWESNGQPENNYKTTPPVLDSDGS FFLYSKLTVDKSRWQQGNVFSCSVMHEALHNHYTQ**E**SLSLSP G | Fc (G, K439E) |
| 30 | GGPSVFLFPPKPKDTLMISRTPEVTCVVVDVSHEDPEVKFNW YVDGVEVHNAKTKPREEQYNSTYRVVSVLTVLHQDWLNGK EYKCKVSNKALPAPIEKTISKAKGQPREPQVYTLPPSREEMTK NQVSLTCLVKGFYPSDIAVEWESNGQPENNYKTTPPVLDSDG SFFLYSKLTVDKSRWQQGNVFSCSVMHEALHNHYTQ**E**SLSLS PG | Fc (GG, K439E) |
| 31 | E**AA**GGPSVFLFPPKPKDTLMISRTPEVTCVVVDVSHEDPEVKF NW**Y**VDGVEVHNAKTKPREEQYNSTYRVVSVLTVLHQDWLN GKEYKCKVSNKALPAPIEKTISKAKGQPREPQVYTLPPSREEM TKNQVSLTCLVKGFYPSDIAVEWESNGQPENNYKTTPPVLDS DGSFFLYSKLTVDKSRWQQGNVFSCSVMHEALHNHYTQ**E**SL SLSPG | Fc (AA, K439E) |
| 32 | APE**AA**GGPSVFLFPPKPKDTLMISRTPEVTCVVVDVSHEDPEV KFNW**Y**VDGVEVHNAKTKPREEQYNSTYRVVSVLTVLHQDW LNGKEYKCKVSNKALPAPIEKTISKAKGQPREPQVYTLPPSRE EMTKNQVSLTCLVKGFYPSDIAVEWESNGQPENNYKTTPPVL DSDGSFFLYSKLTVDKSRWQQGNVFSCSVMHEALHNHYTQ**E** SLSLSPG | Fc (APEAA , K439E) |
| 33 | GPSVFLFPPKPKDTLMISRTPEVTCVVVDVSHEDPEVKFNWY VDGVEVHNAKTKPREEQYNSTYRVVSVLTVLHQDWLNGKE YKCKVSNKALPAPIEKTISKAKGQPREPQVYTLPPSREEMTKN QVSLTCLVKGFYPSDIAVEWESNGQPENNYKTTPPVLDSDGS FFLYSKLTVDKSRWQQGNVFSCSVMHEALHNHYTQ**D**SLSLSP G | Fc (G, K439D) |
| 34 | GGPSVFLFPPKPKDTLMISRTPEVTCVVVDVSHEDPEVKFNW YVDGVEVHNAKTKPREEQYNSTYRVVSVLTVLHQDWLNGK EYKCKVSNKALPAPIEKTISKAKGQPREPQVYTLPPSREEMTK NQVSLTCLVKGFYPSDIAVEWESNGQPENNYKTTPPVLDSDG SFFLYSKLTVDKSRWQQGNVFSCSVMHEALHNHYTQ**D**SLSLS PG | Fc (GG, K439D) |
| 35 | E**AA**GGPSVFLFPPKPKDTLMISRTPEVTCVVVDVSHEDPEVKF NW**Y**VDGVEVHNAKTKPREEQYNSTYRVVSVLTVLHQDWLN GKEYKCKVSNKALPAPIEKTISKAKGQPREPQVYTLPPSREEM TKNQVSLTCLVKGFYPSDIAVEWESNGQPENNYKTTPPVLDS DGSFFLYSKLTVDKSRWQQGNVFSCSVMHEALHNHYTQ**D**SL SLSPG | Fc (AA, K439D) |
| 36 | APE**AA**GGPSVFLFPPKPKDTLMISRTPEVTCVVVDVSHEDPEV KFNW**Y**VDGVEVHNAKTKPREEQYNSTYRVVSVLTVLHQDW LNGKEYKCKVSNKALPAPIEKTISKAKGQPREPQVYTLPPSRE EMTKNQVSLTCLVKGFYPSDIAVEWESNGQPENNYKTTPPVL DSDGSFFLYSKLTVDKSRWQQGNVFSCSVMHEALHNHYTQ**D** SLSLSPG | Fc (APEAA K439D) |
| 37 | GPSVFLFPPKPKDTLMISRTPEVTCVVVDVSHEDPEVKFNWY VDGVEVHNAKTKPREEQYNSTYRVVSVLTVLHQDWLNGKE YKCKVSNKALPAPIEKTISKAKGQPREPQVYTLPPSR**K**EMTKN QVSLTCLVKGFYPSDIAVEWESNGQPENNYKTTPPVLDSDGS FFLYSKLTVDKSRWQQGNVFSCSVMHEALHNHYTQKSLSLSP G | Fc (G, E356K) |

(continued)

| SEQ ID NO: | Amino Acid Sequence | Note |
|---|---|---|
| 38 | GGPSVFLFPPKPKDTLMISRTPEVTCVVVDVSHEDPEVKFNW YVDGVEVHNAKTKPREEQYNSTYRVVSVLTVLHQDWLNGK EYKCKVSNKALPAPIEKTISKAKGQPREPQVYTLPPSR**K**EMTK NQVSLTCLVKGFYPSDIAVEWESNGQPENNYKTTPPV**L**DSDG SFF**L**YSKLTVDKSRWQQGNVFSCSVMHEALHNHYTQKSLSLS PG | Fc (GG, E356K) |
| 39 | E**AA**GGPSVFLFPPKPKDTLMISRTPEVTCVVVDVSHEDPEVKF N**W**YVDGVEVHNAKTKPREEQYNSTYRVVSVLTVLHQDWLN GKEYKCKVSNKALPAPIEKTISKAKGQPREPQVYTLPPSR**K**EM TKNQVSLTCLVKGFYPSDIAVEWESNGQPENNYKTTPPV**L**DS DGSFF**L**YSKLTVDKSRWQQGNVFSCSVMHEALHNHYTQKSL SLSPG | Fc (AA, E356K) |
| 40 | APE**AA**GGPSVFLFPPKPKDTLMISRTPEVTCVVVDVSHEDPEV KFN**W**YVDGVEVHNAKTKPREEQYNSTYRVVSVLTVLHQDW LNGKEYKCKVSNKALPAPIEKTISKAKGQPREPQVYTLPPSR**K** EMTKNQVSLTCLVKGFYPSDIAVEWESNGQPENNYKTTPPV**L** DSDGSFF**L**YSKLTVDKSRWQQGNVFSCSVMHEALHNHYTQK SLSLSPG | Fc (APEAA , E356K) |
| 41 | GPSVFLFPPKPKDTLMISRTPEVTCVVVDVSHEDPEVKFNWY VDGVEVHNAKTKPREEQYNSTYRVVSVLTVLHQDWLNGKE YKCKVSNKALPAPIEKTISKAKGQPREPQVYTLPPSR**R**EMTKN QVSLTCLVKGFYPSDIAVEWESNGQPENNYKTTPPV**L**DSDGS FF**L**YSKLTVDKSRWQQGNVFSCSVMHEALHNHYTQKSLSLSP G | Fc (G, E356R) |
| 42 | GGPSVFLFPPKPKDTLMISRTPEVTCVVVDVSHEDPEVKFNW YVDGVEVHNAKTKPREEQYNSTYRVVSVLTVLHQDWLNGK EYKCKVSNKALPAPIEKTISKAKGQPREPQVYTLPPSR**R**EMTK NQVSLTCLVKGFYPSDIAVEWESNGQPENNYKTTPPV**L**DSDG SFF**L**YSKLTVDKSRWQQGNVFSCSVMHEALHNHYTQKSLSLS PG | Fc (GG, E356R) |
| 43 | E**AA**GGPSVFLFPPKPKDTLMISRTPEVTCVVVDVSHEDPEVKF N**W**YVDGVEVHNAKTKPREEQYNSTYRVVSVLTVLHQDWLN GKEYKCKVSNKALPAPIEKTISKAKGQPREPQVYTLPPSR**R**EM TKNQVSLTCLVKGFYPSDIAVEWESNGQPENNYKTTPPV**L**DS DGSFF**L**YSKLTVDKSRWQQGNVFSCSVMHEALHNHYTQKSL SLSPG | Fc (AA, E356R) |
| 44 | APE**AA**GGPSVFLFPPKPKDTLMISRTPEVTCVVVDVSHEDPEV KFN**W**YVDGVEVHNAKTKPREEQYNSTYRVVSVLTVLHQDW LNGKEYKCKVSNKALPAPIEKTISKAKGQPREPQVYTLPPSR**R** EMTKNQVSLTCLVKGFYPSDIAVEWESNGQPENNYKTTPPV**L** DSDGSFF**L**YSKLTVDKSRWQQGNVFSCSVMHEALHNHYTQK SLSLSPG | Fc (APEAA , E356R) |
| 45 | APE**AA**GGPSVFLFPPKPKDTLMISRTPEVTCVVVDVS**Q**EDPEV **Q**FN**W**YVDGVEVHNAKTKPREE**F**NSTYRVVSVLTVLHQDW LNGKEYKCKVSNK**G**LPS**SI**EKTISKAKGQPREPQVYTLPPS**Q**Q EMTKNQVSLTCLVKGFYPSDIAVEWESNGQPENNYKTTPPV**L** DSDGSFF**L**YS**R**LTVDKSRWQ**E**GNVFSCSVMHEALHNHYTQ**Q** SLSLS**L**G | Fc (APEAA , E356Q, K439Q) |
| Note: In Table 1, the numbering of amino acid positions is in accordance with the EU numbering system. | | |

In another embodiment, the Fc variant comprises an amino acid sequence having at least 85%, 90%, 95% or 99% sequence identity to one of the following sequences: SEQ ID NO: 1-22, SEQ ID NO: 27, SEQ ID NO: 29-45.
Further, the GDF15 active domain is a full-length mature GDF15 protein, a mature GDF15 protein, an N-terminal truncated GDF15 protein or any variant that retains the biological activity of GDF15.

In another embodiment, the GDF15 active domain comprises an amino acid sequence selected from one of the following group, or having at least 85%, 90%, 95% or 99% sequence identity to one of the following group:

SEQ ID NO: 46;

1-14 amino acid truncations at the N-terminal of SEQ ID NO:46, and/or 1-3 amino acid substitutions in SEQ ID NO:46.

In another embodiment, the GDF15 active domain comprises an amino acid sequence selected from one of following group:
SEQ ID NO: 46;
1-14 amino acid truncations at the N-terminal of SEQ ID NO:46, and/or 1-3 amino acid substitutions in SEQ ID NO:46.

Further, the number of amino acid truncations at the N-terminal of SEQ ID NO: 46 is 1, 2, 3, 4, 5, 6, 7, 8, 9, 10, 11, 12, 13 or 14.

Further, the position of amino acid substitution in SEQ ID NO:46 is selected from one, two or three of the following group: position 5, position 6, position 21, position 26, position 30, position 47, position 54, position 55, position 57, position 67, position 69, position 81, position 94, position 107.

Further, the amino acid substitution in the SEQ ID NO: 46 is selected from one, any two of the different positions, or any three of the different positions: D5E, H6D, H6E, R21Q, R21H, D26E, A30S, A47D, A54S, A55E, M57T, R67Q, K69R, A81S, T94E, K107Q.

In some embodiments, the GDF15 active domain comprises the amino acid sequence shown in SEQ ID NO:46.

In other embodiments, the GDF15 active domain comprises the following sequence: an amino acid sequence truncated by 1-14 amino acids at the N-terminal of SEQ ID NO:46.

In still other embodiments, the GDF15 active domain comprises the following sequence: an amino acid sequence with 1-3 amino acid substitutions in SEQ ID NO: 46; the amino acid substitutions in SEQ ID NO: 46 have the meanings described in the present invention.

In yet other embodiments, the GDF15 active domain comprises the following sequence: an amino acid sequence with 1-14 amino acid truncations at the N-terminal of SEQ ID NO: 46 and 1-3 amino acid substitutions in SEQ ID NO: 46; the amino acid substitutions in SEQ ID NO: 46 have the meanings described in the present invention.

In a certain embodiment, the GDF15 active domain comprises one of the following sequences: SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66.

Table 2 GDF15 active domain Sequence

| SEQ ID NO | Amino Acid Sequence | Note |
|---|---|---|
| 46 | ARNGDHCPLGPGRCCRLHTVRASLEDLGWADWVLSPREVQVT MCIGACPSQFRAANMHAQIKTSLHRLKPDTVPAPCCVPASYNPM VLIQKTDTGVSLQTYDDLLAKDCHCI | WT |
| 47 | GDHCPLGPGRCCRLHTVRASLEDLGWADWVLSPREVQVTMCIG ACPSQFRAANMHAQIKTSLHRLKPDTVPAPCCVPASYNPMVLIQ KTDTGVSLQTYDDLLAKDCHCI | ΔN3 |
| 48 | DHCPLGPGRCCRLHTVRASLEDLGWADWVLSPREVQVTMCIGA CPSQFRAANMHAQIKTSLHRLKPDTVPAPCCVPASYNPMVLIQK TDTGVSLQTYDDLLAKDCHCI | ΔN4 |
| 49 | CRLHTVRASLEDLGWADWVLSPREVQVTMCIGACPSQFRAANM HAQIKTSLHRLKPDTVPAPCCVPASYNPMVLIQKTDTGVSLQTYD DLLAKDCHCI | ΔN14 |
| 50 | EHCPLGPGRCCRLHTVRASLEDLGWADWVLSPREVQVTMCIGA CPSQFRAANMHAQIKTSLHRLKPDTVPAPCCVPASYNPMVLIQK TDTGVSLQTYDDLLAKDCHCI | ΔN4, D5E |

(continued)

| SEQ ID NO | Amino Acid Sequence | Note |
|---|---|---|
| 51 | **D**CPLGPGRCCRLHTVRASLEDLGWADWVLSPREVQVTMCIGAC PSQFRAANMHAQIKTSLHRLKPDTVPAPCCVPASYNPMVLIQKT DTGVSLQTYDDLLAKDCHCI | ΔN5, H6D |
| 52 | D**E**CPLGPGRCCRLHTVRASLEDLGWADWVLSPREVQVTMCIGA CPSQFRAANMHAQIKTSLHRLKPDTVPAPCCVPASYNPMVLIQK TDTGVSLQTYDDLLAKDCHCI | ΔN4, H6E |
| 53 | **E**CPLGPGRCCRLHTVRASLEDLGWADWVLSPREVQVTMCIGAC PSQFRAANMHAQIKTSLHRLKPDTVPAPCCVPASYNPMVLIQKT DTGVSLQTYDDLLAKDCHCI | ΔN5, H6E |
| 54 | DHCPLGPGRCCRLHTV**Q**ASLEDLGWADWVLSPREVQVTMCIGA CPSQFRAANMHAQIKTSLHRLKPDTVPAPCCVPASYNPMVLIQK TDTGVSLQTYDDLLAKDCHCI | ΔN4, R21Q |
| 55 | DHCPLGPGRCCRLHTV**H**ASLEDLGWADWVLSPREVQVTMCIGA CPSQFRAANMHAQIKTSLHRLKPDTVPAPCCVPASYNPMVLIQK TDTGVSLQTYDDLLAKDCHCI | ΔN4, R21H |
| 56 | DHCPLGPGRCCRLHTVRASLE**E**LGWADWVLSPREVQVTMCIGA CPSQFRAANMHAQIKTSLHRLKPDTVPAPCCVPASYNPMVLIQK TDTGVSLQTYDDLLAKDCHCI | ΔN4, D26E |
| 57 | DHCPLGPGRCCRLHTVRASLE**E**LGWADWVLSPREVQVTMCIGA CPSQFRAANMHAQIKTSLHRL**R**PDTVPAPCCVPASYNPMVLIQK TDTGVSLQTYDDLLAKDCHCI | ΔN4, D26E, K69R |
| 58 | DHCPLGPGRCCRLHTVRASLEDLGW**S**DWVLSPREVQVTMCIGA CPSQFRAANMHAQIKTSLHRLKPDTVPAPCCVPASYNPMVLIQK TDTGVSLQTYDDLLAKDCHCI | ΔN4, A30S |
| 59 | DHCPLGPGRCCRLHTVRASLEDLGWADWVLSPREVQVTMCIG**D** CPSQFRAANMHAQIKTSLHRLKPDTVPAPCCVPASYNPMVLIQ**K** TDTGVSLQTYDDLLAKDCHCI | ΔN4, A47D |
| 60 | DHCPLGPGRCCRLHTVRASLEDLGWADWVLSPREVQVTMCIGA CPSQFR**S**ANMHAQIKTSLHRLKPDTVPAPCCVPASYNPMVLIQKT DTGVSLQTYDDLLAKDCHCI | ΔN4, A54S |
| 61 | DHCPLGPGRCCRLHTVRASLEDLGWADWVLSPREVQVTMCIGA CPSQFRA**E**NMHAQIKTSLHRLKPDTVPAPCCVPASYNPMVLIQKT DTGVSLQTYDDLLAKDCHCI | ΔN4, A55E |
| 62 | DHCPLGPGRCCRLHTVRASLEDLGWADWVLSPREVQVTMCIGA CPSQFRAAN**T**HAQIKTSLHRLKPDTVPAPCCVPASYNPMVLIQKT DTGVSLQTYDDLLAKDCHCI | ΔN4, M57T |
| 63 | DHCPLGPGRCCRLHTVRASLEDLGWADWVLSPREVQVTMCIGA CPSQFRAANMHAQIKTSLH**Q**LKPDTVPAPCCVPASYNPMVLIQK TDTGVSLQTYDDLLAKDCHCI | ΔN4, R67Q |
| 64 | DHCPLGPGRCCRLHTVRASLEDLGWADWVLSPREVQVTMCIGA CPSQFRAANMHAQIKTSLHRLKPDTVPAPCCVP**S**SYNPMVLIQKT DTGVSLQTYDDLLAKDCHCI | ΔN4, A81S |
| 65 | DHCPLGPGRCCRLHTVRASLEDLGWADWVLSPREVQVTMCIGA CPSQFRAANMHAQIKTSLHRLKPDTVPAPCCVPASYNPMVLIQK TD**E**GVSLQTYDDLLAKDCHCI | ΔN4, T94E |

(continued)

| SEQ ID NO | Amino Acid Sequence | Note |
|---|---|---|
| 66 | DHCPLGPGRCCRLHTVRASLEDLGWADWVLSPREVQVTMCIGA CPSQFRAANMHAQIKTSLHRLKPDTVPAPCCVPASYNPMVLIQK TDTGVSLQTYDDLLA**Q**DCHCI | ΔN4, K107Q |
| Note: In Table 2, the numbering method for indicating amino acid positions is as follows: the first amino acid at the N-terminal of SEQ ID NO: 46 is the first position, and the numbers are sequentially numbered in the direction of the C-terminal. | | |

In another embodiment, the GDF15 active domain comprises an amino acid sequence having at least 85%, 90%, 95% or 99% sequence identity to one of the following sequences: SEQ ID NO: 46-66.

In another embodiment, the GDF 15 active domain comprises an amino acid sequence of one of the following group, or an amino acid sequence having at least 95% sequence identity to one of the following group: SEQ ID NO: 46-66; preferably, the GDF15 active domain comprises an amino acid sequence of one of the following group: SEQ ID NO: 46-66.

In some embodiments, the C-terminal of the Fc variant is linked to the N-terminal of the GDF 15 active domain via a peptide linker; the peptide linker is selected from one or a combination of flexible peptide linkers, rigid peptide linkers. Any suitable linker can be used in the fusion protein of embodiments of the present invention. As herein, the term "linker" refers to a linking moiety containing a peptide linker. Preferably, the linker helps to ensure proper folding, minimize steric hindrance, and does not significantly interfere with the structure of each functional component within the fusion protein. More preferably, in order to obtain higher protein expression yield, rigid peptide linkers and rigid/flexible hybrid peptide linkers were used in the present invention.

In some embodiments, the peptide linker comprises one or a combination of two or more of $(G_4X)_n$, $(X'P)_m$, $(EAAAK)_p$ and $G_q$, wherein each of n, m, p, q is independently selected from an integer of 1-10. X is serine (S) or alanine (A), and X' is alanine (A), lysine (K) or glutamic acid (E).

In a certain embodiment, the peptide linker comprises a combination of $(G_4X)_n$ and $(X'P)_m$, wherein n, m, X, X' have the meanings described in the present invention.

In a certain embodiment, the peptide linker comprises $(G_4X)_{n1}$-$(X'P)_m$-$(G_4X)_{n2}$, wherein each of n1, m, n2 is independently selected from an integer of 1-10. X is S or A, and X' is A, K or E.

In a certain embodiment, the peptide linker comprises $(G_4X)_{n1}$-$(X'P)_m$-$(G_4X)_{n2}$, wherein n1 is 2, m is 10, and n2 is 2.

In another embodiment, the peptide linker comprises a combination of $G_q$ and $(X'P)_m$, wherein q, m, X' having the meanings described herein.

In a certain embodiment, the peptide linker comprises $G_{q1}$-$(X'P)_m$-$G_{q2}$, wherein each of q1, m and q2 is independently selected from an integer of 1-10, and X' is A, K or E.

In a certain embodiment, the peptide linker comprises $G_{q1}$-$(X'P)_m$-$G_{q2}$, wherein q1 is 4, m is 10, and q2 is 4.

In a certain embodiment, the peptide linker comprises a combination of $(G_4X)_n$, $(X'P)_m$ and $G_q$, wherein n, m, q, X, X' have the meanings described in the present invention.

In some embodiments, the peptide linker comprises $(G_4X)_{n1}$-$G_{q1}$-$(X'P)_m$-$(G_4X)_{n2}$-$G_{q2}$, wherein each of n1, q1, m, n2, q2 is independently selected from an integer of 1-10. X is S or A, and X' is A, K or E.

In some embodiments, the peptide linker comprises $(G_4X)_{n1}$-$G_{q1}$-$(X'P)_m$-$(G_4X)_{n2}$-$G_{q2}$, wherein n1 is 1, q1 is 4, m is 10, n2 is 1, and q2 is 4.

In some embodiments, the peptide linker comprises $(G_4S)_5$ (SEQ ID NO: 123); in some embodiments, the peptide linker comprises $(G_4S)_8$ (SEQ ID NO: 124); in some embodiments of the present invention, the peptide linker comprises $G_4(AP)_{10}G_4$ (SEQ ID NO: 125); in other embodiments of the present invention, the peptide linker comprises $(G_4S)_2(AP)_{10}(G_4S)_2$ (SEQ ID NO: 126); in still other embodiments of the present invention, the peptide linker comprises $(G_4S)_2(EP)_{10}(G_4S)_2$ (SEQ ID NO: 127). After extensive research, the inventors found that the use of rigid peptide linkers or rigid/flexible hybrid peptide linkers can significantly increase the expression of the fusion protein of the present invention.

In some embodiments, the fusion protein comprises an amino acid sequence selected from one of the following group: SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, SEQ ID NO: 91, SEQ ID NO: 92, SEQ ID NO: 93, SEQ ID NO: 94, SEQ ID NO: 95, SEQ ID NO: 96, SEQ ID NO: 97, SEQ ID NO: 98, SEQ ID NO: 99, SEQ ID NO: 100, SEQ ID NO: 101, SEQ ID NO: 102, SEQ ID NO: 105, SEQ ID NO: 106, SEQ ID NO: 107, SEQ ID NO: 108, SEQ ID NO: 109, SEQ ID NO: 110, SEQ ID NO: 111, SEQ ID NO: 112, SEQ ID NO: 113, SEQ ID NO: 114, SEQ ID NO: 115, SEQ ID NO: 116, SEQ ID NO: 117, SEQ ID NO: 118.

Table 3 Fusion protein

| SEQ ID NO: | Name | Fc variant sequenc e SEQ ID NO: | Note of mutation in Fc variant | Sequence of GDF 15 active domain SEQ ID NO: | Note of GDF active domain |
|---|---|---|---|---|---|
| 119 | M1 | 25 | Fc (APEAA) | 48 | ΔN4 |
| 67 | M2 | 4 | Fc (APEAA, K439E) | 46 | WT |
| 68 | M3 | 4 | Fc (APEAA, K439E) | 47 | ΔN3 |
| 69 | M4 | 4 | Fc (APEAA, K439E) | 48 | ΔN4 |
| 70 | M5 | 8 | Fc (APEAA, K439D) | 48 | ΔN4 |
| 71 | M6 | 17 | Fc (APEAA, K439Q) | 48 | ΔN4 |
| 72 | M7 | 19 | Fc (APEAA, K439A) | 48 | ΔN4 |
| 73 | M8 | 12 | Fc (APEAA, E356K) | 48 | ΔN4 |
| 74 | M9 | 16 | Fc (APEAA, E356R) | 48 | ΔN4 |
| 75 | M10 | 20 | Fc (APEAA, E356Q) | 48 | ΔN4 |
| 76 | M11 | 22 | Fc (APEAA, E356A) | 48 | ΔN4 |
| 77 | M12 | 32 | Fc (APEAA, K439E) | 48 | ΔN4 |
| 78 | M13 | 3 | Fc (AA, K439E) | 48 | ΔN4 |
| 79 | M14 | 26 | Fc (GG) | 48 | ΔN4 |
| 80 | M15 | 2 | Fc (GG, K439E) | 48 | ΔN4 |
| 81 | M16 | 1 | Fc (G, K439E) | 48 | ΔN4 |
| 82 | M17 | 2 | Fc (GG, K439E) | 48 | ΔN4 |
| 83 | M18 | 2 | Fc (GG, K439E) | 48 | ΔN4 |
| 84 | M19 | 2 | Fc (GG, K439E) | 48 | ΔN4 |
| 85 | M20 | 4 | Fc (APEAA, K439E) | 50 | ΔN4, D5E |
| 86 | M21 | 4 | Fc (APEAA, K439E) | 51 | ΔN5, H6D |
| 87 | M22 | 4 | Fc (APEAA, K439E) | 52 | ΔN4, H6E |
| 88 | M23 | 4 | Fc (APEAA, K439E) | 53 | ΔN5, H6E |
| 89 | M24 | 4 | Fc (APEAA, K439E) | 54 | ΔN4, R21Q |
| 90 | M25 | 4 | Fc (APEAA, K439E) | 55 | ΔN4, R21H |
| 91 | M26 | 4 | Fc (APEAA, K439E) | 56 | ΔN4, D26E |
| 92 | M27 | 4 | Fc (APEAA, K439E) | 57 | ΔN4, D26E, K69R |
| 93 | M28 | 4 | Fc (APEAA, K439E) | 58 | ΔN4, A30S |
| 94 | M29 | 4 | Fc (APEAA, K439E) | 59 | ΔN4, A47D |
| 95 | M30 | 4 | Fc (APEAA, K439E) | 60 | ΔN4, A54S |
| 96 | M31 | 4 | Fc (APEAA, K439E) | 61 | ΔN4, A55E |
| 97 | M32 | 4 | Fc (APEAA, K439E) | 62 | ΔN4, M57T |
| 98 | M33 | 4 | Fc (APEAA, K439E) | 63 | ΔN4, R67Q |
| 99 | M34 | 4 | Fc (APEAA, K439E) | 64 | ΔN4, A81S |
| 100 | M35 | 4 | Fc (APEAA, K439E) | 65 | ΔN4, T94E |

(continued)

| SEQ ID NO: | Name | Fc variant sequence SEQ ID NO: | Note of mutation in Fc variant | Sequence of GDF 15 active domain SEQ ID NO: | Note of GDF active domain |
|---|---|---|---|---|---|
| 101 | M36 | 4 | Fc (APEAA, K439E) | 66 | ΔN4, K107Q |
| 102 | M37 | 4 | Fc (APEAA, K439E) | 49 | ΔN14 |
| 103 | M38 | 28 | Fc(GG,F405Q/Y407E, K439E) | 48 | ΔN3 |
| 104 | monoF c-GDF 15 | 24 | Fc (AA, F405Q/Y407E) | 48 | ΔN3 |
| 105 | M39 | 17 | Fc (APEAA, K439Q) | 47 | ΔN3 |
| 106 | M40 | 20 | Fc (APEAA, E356Q) | 47 | ΔN3 |
| 107 | M41 | 18 | Fc (APEAA, K439N) | 47 | ΔN3 |
| 108 | M42 | 21 | Fc (APEAA, E356N) | 47 | ΔN3 |
| 109 | M43 | 19 | Fc (APEAA, K439A) | 47 | ΔN3 |
| 110 | M44 | 22 | Fc (APEAA, E356A) | 47 | ΔN3 |
| 111 | M45 | 17 | Fc (APEAA, K439Q) | 47 | ΔN3 |
| 112 | M46 | 4 | Fc (APEAA, K439E) | 47 | ΔN3 |
| 113 | M47 | 8 | Fc (APEAA, K439D) | 47 | ΔN3 |
| 114 | M48 | 12 | Fc (APEAA, E356K) | 47 | ΔN3 |
| 115 | M49 | 16 | Fc (APEAA, E356R) | 47 | ΔN3 |
| 116 | M50 | 45 | Fc (APEAA, E356Q, K439Q) | 47 | ΔN3 |
| 117 | M51 | 18 | Fc (APEAA, K439N) | 48 | ΔN4 |
| 118 | M52 | 21 | Fc (APEAA, E356N) | 48 | ΔN4 |
| 120 | M53 | 2 | Fc (GG, K439E) | 48 | ΔN4 |
| 121 | M54 | / | Fc (APEAA, E356D) | 48 | ΔN4 |
| 122 | M55 | / | Fc (APEAA, K439R) | 48 | ΔN4 |

Note: In Table 3, the numbering of the amino acid positions in the note of mutation in Fc variant follows the EU numbering; the numbering method for indicating amino acid positions in the GDF15 active domain is as follows: the first amino acid at the N-terminal of SEQ ID NO: 46 is the first position, and the numbers are sequentially numbered in the direction of the C-terminal.

In another embodiment, the fusion protein comprises an amino acid sequence having at least 85%, 90%, 95% or 99% sequence identity to one of the following sequences: SEQ ID NO: 67-78, SEQ ID NO: 80-102, SEQ ID NO: 105-118. In another embodiment, the fusion protein comprises an amino acid sequence of one of the following group, or an amino acid sequence having at least 95% sequence identity to one of the following group: SEQ ID NO: 67-78, SEQ ID NO: 80-102, SEQ ID NO: 105-118; preferably, the fusion protein comprises an amino acid sequence of one of the following group: SEQ ID NO: 67-78, SEQ ID NO: 80-102, SEQ ID NO: 105-118.

In the second aspect, the present invention provides a method for treating obesity-related diseases using a homodimer fusion protein, which is composed of the fusion protein described in the first aspect of the present invention.

In the third aspect, the present invention provides a method for treating obesity-related diseases using a biological material, wherein the biological material is any one of (i), (ii), and (iii):

(i) a nucleic acid comprising a nucleotide sequence encoding the fusion protein of the present invention;
(ii) a vector comprising the nucleic acid of (i);
(iii) a host cell containing the nucleic acid of (i) and/or the vector of (ii).

In the fourth aspect, the present invention provides a method for treating obesity-related diseases using a pharmaceutical composition comprising the homodimeric fusion protein of the present invention as an active ingredient, wherein the homodimeric fusion protein is present in the pharmaceutical composition in a therapeutically effective amount.

Further, the pharmaceutical composition also comprises a pharmaceutically acceptable carrier.

Compared with the prior art, the present invention has the following advancements: compared with the fusion protein of unmutated or monomeric or heterodimeric IgG Fc and GDF15 active domain, by fusing the Fc variant provided by the present invention with the any GDF15 domain that retains biological activity (mature GDF15, truncated GDF15, or variants thereof), the Fc-GDF15 fusion protein has significantly improved physicochemical properties and recombinant expression levels, has *in vitro* activity equivalent to or better than natural GDF15 molecules, and has significantly prolonged *in vivo* circulating half-life, which can support the dosing frequency of once every two weeks or even once a month when treating obesity. In addition, the Fc-GDF15 fusion protein of the present invention has simpler manufacturing process and lower cost of production than fusion proteins formed by heterodimers in the prior art, and better patient compliance.

## DESCRIPTION OF THE DRAWINGS

Various other advantages and benefits will become apparent to those of ordinary skill in the art upon reading the following detailed description of the preferred embodiments. The drawings are for the purpose of illustrating preferred embodiments only and are not to be considered limiting of the invention. In the drawings:

Figure 1: Schematic diagram of the homodimer formed by the Fc-GDF15 fusion protein;

Figure 2: Effect of single-dose administration of Fc-GDF15 fusion proteins on body weight in normal mice (monitored for 14 days);

Figure 3: Effect of single-dose administration of Fc-GDF15 fusion proteins on food intake in normal mice (monitored for 14 days);

Figure 4: Effect of single-dose administration of Fc-GDF15 fusion proteins on body weight in normal mice (monitored for 30 days);

Figure 5: Effect of single-dose administration of Fc-GDF15 fusion proteins on food intake in normal mice (monitored for 30 days);

Figure 6: Effect of single-dose administration of Fc-GDF15 fusion proteins on body weight in normal mice (monitored for 56 days);

Figure 7: Effect of single-dose administration of Fc-GDF15 fusion proteins on food intake in normal mice (monitored for 56 days);

Figure 8: Effect of repeat-dose administration of Fc-GDF15 fusion proteins on body weight in diet-induced obese mice (monitored for 42 days);

Figure 9: Result of glucose tolerance test in diet-induced obese mice after repeat-dose administration of Fc-GDF15 fusion proteins;

Figure 10: Effect of repeat-dose administration of M39 construct at different dose levels on body weight in diet-induced obese mice (monitored for 49 days);

Figure 11: Fasting blood glucose level in diet-induced obese mice after repeat-dose administration of different doses of M39 construct (on the 46th day) (***-$p < 0.001$ vs. vehicle. Statistical analysis method: one-way ANOVA, followed by Dunnett's);

Figure 12: a. Result of glucose tolerance test in diet-induced obese mice after repeat-dose administration of different dose levels of M39 construct (on the 46th day); b. Statistics of area under the glucose tolerance test curve (**-$p < 0.01$, ***-$p < 0.001$ vs. vehicle. Statistical analysis method: one-way ANOVA, followed by Dunnett's);

Figure 13: Fat index in diet-induced obese mice after repeat-dose administration of different doses of M39 construct (**-$p < 0.01$, ***-$p < 0.001$, #-$p < 0.05$, ##-$p < 0.01$, ###-$p < 0.001$ vs. vehicle. Statistical analysis method: one-way ANOVA, followed by Dunnett's);

Figure 14: Alanine transaminase level in diet-induced obese mice after repeat-dose administration of different doses of M39 construct (***-$p < 0.001$ vs. vehicle. Statistical analysis method: one-way ANOVA, followed by Dunnett's);

Figure 15: Aspartate transaminase level in diet-induced obese mice after repeat-dose administration of different doses of M39 construct (***-$p < 0.001$ vs. vehicle. Statistical analysis method: one-way ANOVA, followed by Dunnett's);

Figure 16: Effect of repeat-dose administration of M6 and M39 constructs at different doses on body weight in ob/ob obese mice (monitored for 52 days). a. Body weight change relative to baseline; b. Body weight change relative to vehicle control;

Figure 17 Effect of repeat-dose administration of M6 and M39 constructs on food intake in ob/ob obese mice at different doses (monitored for 52 days);

Figure 18: Liver index in ob/ob obese mice after repeat-dose administration of different doses of M6 and M39 constructs (***-p<0.001 vs. vehicle, ###-p<0.001 vs. Semaglutide. Statistical analysis method: one-way ANOVA, followed by Dunnett's);

Figure 19: Alanine transaminase level in ob/ob obese mice after repeat-dose administration of different doses of M6 and M39 constructs (***-p<0.001 vs. vehicle, ##-p<0.01 vs. Semaglutide. Statistical analysis method: one-way ANOVA, followed by Dunnett's);

Figure 20: Aspartate transaminase level in ob/ob obese mice after repeat-dose administration of different doses of M6 and M39 constructs (*-p<0.05, **-p<0.01, ***-p<0.001 vs. vehicle, #-p<0.05 vs. Semaglutide. Statistical analysis method: one-way ANOVA, followed by Dunnett's);

Figure 21: Improvement of liver pathology in ob/ob obese mice after repeat-dose administration of different doses of M6 and M39 constructs (***-p<0.001 vs. vehicle, ###-p<0.001 vs. Semaglutide. Statistical analysis method: one-way ANOVA, followed by Dunnett's).

## EXAMPLES

Exemplary embodiments of the present disclosure will be described in more detail below with reference to the accompanying drawings. While exemplary embodiments of the present disclosure are shown in the drawings, it should be understood that the present disclosure may be embodied in various forms and should not be limited by the embodiments set forth herein. Rather, these embodiments are provided so that the present disclosure will be more thoroughly understood, and will fully convey the scope of the present disclosure to those skilled in the art.

Unless otherwise defined, all technical and scientific terms used in the present invention have the same meanings as commonly understood by one of ordinary skill in the art. In addition to the specific methods, devices and materials used in the embodiments, according to the mastery of the prior art by those skilled in the art and the description of the present invention, any methods, devices and materials in the prior art that are similar or equivalent to the methods, devices and materials described in the embodiments of the present invention can also be used to implement the present invention.

As used herein, the terms "native", "wild-type" or "WT" refer to a protein or polypeptide that does not contain any genetically engineered mutation, that is naturally occurring or that can be isolated from the environment.

As used herein, an amino acid "substitution" or "replacement" refers to the replacement of one amino acid in a polypeptide with another amino acid.

As used herein, the amino acid substitution is indicated by a first letter followed by a number followed by a second letter. The first letter refers to the amino acid in the wild-type protein; the number refers to the amino acid position in which it was substituted; the second letter refers to the amino acid used to replace the wild-type amino acid.

Herein, deletions at the amino terminus of proteins or polypeptides are indicated by $\Delta N$ followed by a number, wherein the number represents the number of amino acids deleted at the amino terminal.

As used herein, "IgG" or "IgG antibody" refers to an antibody having the structure of a naturally occurring immunoglobulin G molecule. IgG antibodies include, for example, IgG1, IgG2, IgG3, and IgG4. IgG1 Fc, IgG2 Fc, IgG3 Fc, IgG4 Fc represent the Fc or Fc region of IgG1, IgG2, IgG3, and IgG4, respectively. The IgG Fc in the fusion protein herein can be IgG1 Fc, IgG2 Fc, IgG3 Fc, IgG4 Fc, preferably IgG1 Fc, IgG4 Fc.

As used herein, a "peptide linker" refers to a single amino acid or polypeptide sequence that joins two proteins together. The length of peptide linkers can be, for example, about 1-40 amino acids, including, for example, repeated alanines, glycines, and serines. Common peptide linkers include flexible peptide linkers, for example, a combination of glycine and serine, such as $(GGGGS)_n$, which can adjust the distance between the connected proteins by the number of repeat units in the peptide linker; peptide linkers composed only of glycine, such as $G_n$, common ones include $G_6$, $G_8$, etc.; rigid peptide linkers, such as $\alpha$-helical peptide linkers with the $(EAAAK)_n$ sequence; peptide linkers with proline-rich $(XP)_n$, wherein X can specify any amino acid, common ones include alanine, lysine, or glutamic acid, $(XP)_n$ sequence has no helical structure, and the presence of proline in the peptide linker can increase backbone stiffness and effectively separate domains. Structures with proline-rich sequences are widespread in the body, such as the structure of $(AP)_7$ at the N-terminal of skeletal muscle proteins. The selection of peptide linkers can be determined by those skilled in the art based on usual experiments, for example, using only flexible peptide linkers, using only rigid peptide linkers, using a combination of flexible peptide linkers and rigid peptide linkers, and the like.

Herein, "Fc" or "Fc region" is used to define the C-terminal region of the heavy chain of an antibody that contains at least a portion of the constant region. The Fc region can be a native sequence Fc region or a variant Fc region. Those skilled in the art know that an Fc region generally comprises two constant domains: CH2 and CH3, and that the boundaries of the Fc region can vary. In this context, the C-terminal of the Fc region is the C-terminal of the heavy chain of the antibody; while the N-terminal of the Fc region may vary. In some embodiments, the N-terminal of the Fc region may start from, for example, position 231 (EU numbering), or position 233 (EU numbering), or position 236 (EU numbering), or position 237 (EU numbering); in other embodiments, the N-terminal of the Fc region of the present invention does not comprise a hinge region; in yet other embodiments, the N-terminal of the Fc region of the present invention comprises a hinge region. Herein, the numbering of amino acid residues in the Fc region is according to the EU numbering system, also known as the EU index, as described in Edelman et al., The covalent structure of an entire $\gamma$G immunoglobulin molecule. Proc. Natl. Acad. Sci., USA, 1969.

GDF15 polypeptide needs to form a dimer to exercise its activity. During the research process of the present invention, it was found that although IgG Fc can naturally form a dimer, the spatial distance between the carboxyl terminal of the two monomer Fc in the IgG Fc dimer is significantly different from that of the amino terminal of the GDF 15 monomer; even if a flexible or rigid peptide linker is used, the fusion protein formed by the C-terminal of IgG Fc and the N-terminal of GDF15 still has issues such as instability and low expression yield during assembly. The Fc variants provided by the present invention significantly improve the stability of the Fc-GDF15 molecule by introducing mutations at specific Fc sites such as position 439 or 356, realizes the efficient assembly of the Fc-GDF15 fusion protein, and increases the expression yield. The manufacturing process is simple, and the fusion proteins retain the ability to form homodimers. In addition, the Fc variants of the present invention can be applied to all GDF15 proteins or variants thereof that retain biological activity, whether it is the mature GDF15 protein, or its truncated GDF15, or any variant thereof, which can form fusion proteins with the Fc variants of the present invention and play a role.

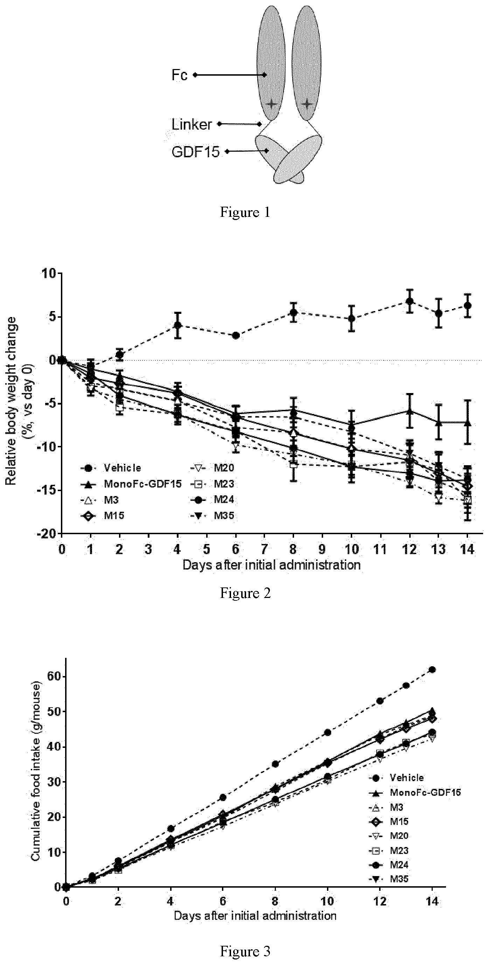

As used herein, "dimer" refers to a protein dimer, which is composed of two protein molecules, and is the quaternary structure of a protein. The two protein molecules that make up the dimer may be referred to as the monomer molecule or monomer protein of the dimer. Dimers can include both "homodimers" and "heterodimers", wherein a homodimer is formed by the combination of two identical monomer molecules; a heterodimer is formed by the combination of two different monomer molecules. It is generally believed that homodimers are simpler to prepare than heterodimers because only one peptide needs to be encoded. In the present invention, the Fc variant provided by the present invention has the ability to form homodimers; the fusion protein of the Fc variant provided by the present invention and the GDF15 active domain also has the ability to form homodimers. Referring to Figure 1, in the homodimer formed by the fusion protein, homodimerization exists between two monomer Fc variants and between two monomer GDF15 active domains, respectively.

Herein, "growth differentiation factor 15", "GDF15" or "GDF15 active domain" in some embodiments is native mature human GDF15 or a domain thereof, and in other embodiments refers to a mutant of GDF 15 or a domain thereof. The mature GDF15 is composed of amino acids 197 (a) to 308 (I) except for signal peptide and leader peptide (GDF15 (197-308) (SEQ ID No: 46)) among a total of 308 amino acids (UniProt q99988), or a polypeptide having at least 85%, 90%, 95%, and 99% sequence identity with the amino acid sequence within the range of maintaining the unique activity and structure of GDF15. The mutant comprises truncations, and/or one or more amino acid mutations. In some embodiments, the truncated GDF15 may be an N-terminal deletion variant, such as a sequence of 1-14 amino acids truncated at the N-terminal, or a polypeptide having at least 85%, 90%, 95%, and 99% sequence identity with the truncated amino acid sequence; In some embodiments, the GDF15 variant refers to the substitution, insertion or deletion of at least one amino acid site compared with the mature GDF15 or the truncated GDF15. As long as it maintains at least one of the biological activities of the GDF15 protein, such as its effects on food intake, blood glucose level, insulin resistance and body weight, *etc.,* are within the scope of protection of the present invention.

The GDF15 variants may also be generated by introducing one or more amino acid substitutions, either conservative or non-conservative and using naturally occurring or non-naturally occurring amino acids, or by deleting specific residues or segments thereof, at particular positions of the GDF15 polypeptide.

In the present application, the term "conservative amino acid substitutions" may involve a substitution of a native amino acid residue (i.e., a residue found in a given position of the wild-type GDF15 polypeptide sequence) with a non-native residue (i.e., a residue that is not found in that same position of the wild-type GDF15 polypeptide sequence) such that there is little or no effect on the polarity or charge of the amino acid residue at that position. Conservative amino acid substitutions also encompass non-naturally occurring amino acid residues (as defined herein) that are typically incorporated by chemical peptide synthesis rather than by synthesis in biological systems. These include peptidomimetics, and other reversed or inverted forms of amino acid moieties.

The naturally occurring amino acid residues can be grouped into the following classes based on common side chain properties:

    (1) Hydrophobic residues: Met, Ala, Val, Leu, Ile;
    (2) Neutral hydrophilic residues: Cys, Ser, Thr, Asn, Gln;
    (3) Acid residues: Asp, Glu;
    (4) Alkaline residues: His, Lys, Arg;
    (5) Residues affecting chain orientation: Gly, Pro; and
    (6) Aromatic residues: Trp, Tyr, Phe.

Conservative substitutions may include one member of these categories being exchanged for another member of the same category.

Some conservative amino acid substitutions are shown in the table below:

| Amino acid | Conservative amino acid substitutions |
|---|---|
| Ala | D-Ala, Gly, Aib, β-Ala, L-Cys, D-Cys |
| Arg | D-Arg, Lys, Orn, D-Orn |
| Asn | D-Asn, Asp, D-Asp, Glu, D-Glu, Gln, D-Gln |
| Asp | D-Asp, D-Asn, Asn, Glu, D-Glu, Gln, D-Gln |
| Cys | D-Cys, S-Me-Cys, Met, D-Met, Thr, D-Thr, L-Ser, D-Ser |
| Gln | D-Gln, Asn, D-Asn, Glu, D-Glu, Asp, D-Asp |
| Glu | D-Glu, Asp, D-Asp, Asn, D-Asn, Gln, D-Gln |
| Gly | Ala, D-Ala, Pro, D-Pro, Aib, β-Ala |
| Ile | D-Ile, Val, D-Val, Leu, D-Leu, Met, D-Met |
| Leu | D-Leu, Val, D-Val, Met, D-Met, Ile, D-Ile |
| Lys | D-Lys, Arg, D-Arg, Orn, D-Orn |
| Met | D-Met, S-Me-Cys, Ile, D-Ile, Leu, D-Leu, Val, D-Val |
| Phe | D-Phe, Tyr, D-Tyr, His, D-His, Trp, D-Trp |
| Pro | D-Pro |
| Ser | D-Ser, Thr, D-Thr, allo-Thr, L-Cys, D-Cys |
| Thr | D-Thr, Ser, D-Ser, allo-Thr, Met, D-Met, Val, D-Val |
| Tyr | D-Tyr, Phe, D-Phe, His, D-His, Tip, D-Trp |
| Val | D-Val, Leu, D-Leu, Ile, D-Ile, Met, D-Met |

A conservative substitution may involve the exchange of a member of one of these categories with another member of the same category. Non conservative substitutions may involve the exchange of members of one of these categories with members of another category.

As used herein, "identity" or "homology" generally refers to the sequence similarity between two peptides or proteins or between two nucleic acid molecules. Percent "identity" or "homology" refers to the percentage of identical residues between amino acids or nucleotides in the molecules being compared, and is calculated based on the size of the smallest of the molecules being compared.

The fusion protein formed by the Fc variant of the present invention and the natural mature human GDF15 or its variant can achieve the purpose of the present invention. As used herein, "vector" refers to a nucleic acid molecule capable of propagating another nucleic acid to which it is linked, including vectors that are self-replicating nucleic acid structures and vectors that integrate into the genome of a host cell into which it is introduced. Certain vectors are capable of directing the expression of nucleic acids to which they are operably linked, and such vectors are referred to herein as "expression vectors".

As used herein, "host cell" refers to a cell into which exogenous nucleic acid has been introduced, including progeny of such cells. Host cells include the initially transformed cell and progeny derived therefrom (regardless of passage number). The progeny may not be identical to the parent cell in the content of nucleic acid, but may contain mutations. Mutant progeny having the same function or biological activity as screened or selected in the original transformed cell are included herein. A host cell is any type of cellular system that can be used to produce the fusion proteins of the present invention. Host cells include mammalian cultured cells such as CHO cells, BHK cells, NS0 cells, SP2/0 cells, YO myeloma cells, P3X63 mouse myeloma cells, PER cells, PER.C6 cells or hybridoma cells, yeast cells, bacterial cells such as E. coli, insect cells and plant cells, etc., and also include cells contained in transgenic animals, transgenic plants, or cultured plant or animal tissue.

As used herein, a "pharmaceutical composition" refers to a formulation in a form that permits the biological activity of the active ingredient contained therein to be effective and free of additional ingredients that would be unacceptably toxic to subjects to whom the pharmaceutical composition would be administered.

As used herein, a "therapeutically effective amount" refers to an amount effective to achieve a desired therapeutic or prophylactic result, including, for example, eliminating, reducing, delaying, minimizing or preventing adverse effects of a disease.

As used herein, a "pharmaceutically acceptable carrier" refers to ingredients other than the active ingredient in the pharmaceutical composition that are not toxic to the subject. Pharmaceutically acceptable carriers include, but are not limited to, buffers, excipients, stabilizers or preservatives.

As used herein, "treatment" refers to an attempt to alter the natural course of disease in a treated individual, and may be for prevention or clinical intervention performed during the course of clinical pathology. Desired effects of treatment include, but are not limited to, preventing the occurrence or recurrence of the disease, alleviating symptoms, reducing any direct or indirect pathological consequences of the disease, slowing the rate of disease progression, improving or eliminating the disease state, and regressing or improving the prognosis.

As used herein, an "individual" or a "subject" is a mammal. Mammals include, but are not limited to, primates (e.g., humans and non-human primates such as monkeys) or other mammals (e.g., cows, sheep, cats, dogs, horses, rabbits, and rodents such as mice and rats). In particular, the individual or subject is a human.

Herein, the conventional one-letter or three-letter codes for natural amino acids are used:

| | |
|---|---|
| Alanine (Ala, A) | Arginine (Arg, R) |
| Asparagine (Asn, N) | Aspartic acid (Asp, D) |
| Cysteine (Cys, C) | Glutamic acid (Glu, E) |
| Glutamine (Gln, Q) | Glycine (Gly, G) |
| Histidine (His, H) | Isoleucine (Ile, I) |
| Leucine (Leu, L) | Lysine (Lys, K) |
| Methionine (Met, M) | Phenylalanine (Phe, F) |
| Proline (Pro, P) | Serine (Ser, S) |
| Threonine (Thr, T) | Tryptophan (Trp, W) |
| Tyrosine (Tyr, Y) | Valine (Val, V) |

**Example 1 Design of Fc variant**

In this example, the effects of introducing different mutations on the structural properties of Fc were tested, and the test results of some mutants are shown in Table 4. Test steps include:

Molecular cloning method was used to insert Fc nucleic acid sequence into mammalian cell expression vector, and ExpiCHO Fectamine™ CHO Transfection Kit (ThermoFisher Scientific) was used for transient transfection and expression in CHO-S cells; after purification by Protein A column, the target protein was obtained, and the target protein was analyzed by size exclusion high performance liquid chromatography (SEC).

The main principle of SEC analysis is based on the molecular weight and three-dimensional structure of the analyte (such as protein). Generally, analytes with larger molecular weights will pass through the chromatographic column more quickly, so they have a shorter column retention time. On the contrary, analytes with smaller molecular weights have longer retention times. So SEC can be used to analyze the aggregation of protein molecules.

The natural immunoglobulin Fc fragment will naturally form a homodimer structure. In order to improve the stability of the fusion protein, the Fc molecule needs to be mutated, which may destroy the structure of Fc homodimer. However, the inventors surprisingly found that the Fc molecule (K439D or K439E) after the introduction of the mutation at position 439 had the same retention time in the SEC analysis as the Fc molecule without mutation, indicating that the introduced mutation did not disrupt the formation of Fc dimer. As a control, this example also tested an Fc molecule (including the F405Q/Y407E mutation) with an amino acid sequence of SEQ ID NO: 24. According to the previous patent document WO2019195091, the F405Q/Y407E mutation can destroy the Fc dimer to form a monomeric Fc molecule. In this experiment, it was found that the mutation had a significantly prolonged retention time, indicating that the Fc molecule did exist in a monomeric form.

Table 4 Properties of the formation of Fc variant dimer

| Sequence | Fc variant | Retention time | Aggregate state |
|---|---|---|---|
| SEQ ID NO: 23 | IgG4 Fc (AA) | 13.341 min | Dimer |
| SEQ ID NO: 3 | IgG4 Fc (AA, K439E) | 13.156 min | Dimer |
| SEQ ID NO: 7 | IgG4 Fc (AA, K439D) | 13.093 min | Dimer |
| SEQ ID NO: 24 | IgG4 Fc (AA, F405Q/Y407E) | 15.076 min | monomer |

**Example 2 Preparation of Fc-GDF15 construct**

Construction of expression vectors: The Fc-GDF15 molecules shown in Table 3 were inserted into the polyclonal restriction sites of the mammalian cell vector pXC17.4, respectively, to construct expression vectors expressing the Fc-GDF15 shown in Table 3; the Fc-GDF15 expression vector was extracted with endotoxin-depleted plasmid extraction kit (OMEGA) for cell transfection.

Cell transfection and culture: CHO-S cells were recovered and subcultured, and the cells were collected when the density reached about $6 \times 10^6$ cells/mL for cell transfection. Cells were transfected using ExpiCHO FectamineTM CHO Transfection Kit (ThermoFisher Scientific), in which the final concentration of Fc-GDF15 expression vector was 1 μg/ml. About 20 hours after transfection, ExpiCHO Fectamine CHO Enhancer and ExpiCHO Feed were added to maintain the growth of transfected cells. The cell culture medium was harvested when the cell viability decreased to about 80%.

Protein purification: After centrifuging the cell culture medium, the supernatant was collected and filtered with a 0.22 μm filter to obtain the cell culture supernatant. The cell culture supernatant was purified using a two-step chromatography method. The first step of chromatography: The cell culture supernatant was applied to an AT Protein A Diamond column (BestChrom) equilibrated with phosphate buffered saline (PBS). After the target protein was bound, the mixture was washed with equilibration solution for more than 3CV (column volume) to remove unbound impurities, and eluted with 100mM acetic acid-sodium acetate (pH 3.0) buffer. The target protein was collected, then the pH of the collected sample was quickly adjusted to 7.2-7.6 with 1.0M Tris-HCl (pH 8.0) solution, and the pH-adjusted sample was diluted with purified water until its conductivity value was lower than 5ms/cm to obtain the first sample. The second step of chromatography: The first sample obtained by the Protein A was further purified using a Bestarose Q HP column, which was equilibrated with 20 mM Tris-HCl buffer. The first sample was loaded, washed with 20 mM Tris-HCl buffer for 3CV and 20mM PB buffer for more than 3CV to replace the buffer system, and finally eluted with PBS. The elution fraction was collected to obtain the target protein sample. The nature and purity of target protein samples were evaluated by sodium dodecyl sulfate polyacrylamide gel electrophoresis (SDS-PAGE) and mass spectrometry analysis. The target protein samples were quantified by a micro-nucleic acid protein analyzer (NanoDrop 2000/2000c Spectrophotometer).

**Example 3 Effect of Fc variants on the expression yield of Fc-GDF15 fusion protein**

The Fc-GDF15 fusion proteins described in Table 3 were expressed by the method described in Example 2, and the effects of applying different Fc variants on the expression yield of Fc-GDF15 fusion proteins were compared. The results of the tests are shown in Table 5.

According to the test results, it was found that the Fc-GDF15 fusion proteins that did not mutate at K439 (EU numbering) or E356 (EU numbering) of Fc, such as M1 (a homodimer with a monomer having the sequence of SEQ ID NO: 119), M14 (a homodimer with a monomer having the sequence of SEQ ID NO: 79), had lower expression yields, while the Fc-GDF15 fusion proteins mutated at K439 and/or E356 of Fc, such as M2 (a homodimer with a monomer having the sequence of SEQ ID NO: 67), M3 (a homodimer with a monomer having the sequence of SEQ ID NO: 68), M4 (a homodimer with a monomer having the sequence of SEQ ID NO: 69), M5 (a homodimer with a monomer having the sequence of SEQ ID NO: 70), M6 (a homodimer with a monomer with the sequence of SEQ ID NO: 71), M7 (a homodimer with a monomer with the sequence of SEQ ID NO: 72), M8 (a homodimer with a monomer having the sequence of SEQ ID NO: 73), M9 (a homodimer with a monomer having the sequence of SEQ ID NO: 74), M10 (a homodimer with a monomer having the sequence of SEQ ID NO: 75), M11 (a homodimer with a monomer having the sequence of SEQ ID NO: 76), M15 (a homodimer with a monomer having the sequence of SEQ ID NO: 80), M39 (a homodimer with a monomer having the sequence of SEQ ID NO: 105), M50 (a homodimer with a monomer having the sequence of SEQ ID NO: 116), had improved fusion protein expression yields, indicating that the mutation of amino acid at position 356 (EU numbering) or 439 (EU numbering) was more favorable for the assembled expression of Fc-GDF15 fusion protein.

Table 5 Expression yield of Fc-GDF15 fusion protein comprising different Fc variants

| Fc-GDF15 Construct | Fc mutation at position 356 or 439 | Expression volume (ml) | Expression yield (Protein A purification, μg/ml culture medium) |
|---|---|---|---|
| M14 | Unmutated | 50 | 34 |
| M15 | K439E | 50 | 252 |
| M1 | Unmutated | 100 | 67 |
| M2 | K439E | 100 | 145 |
| M3 | K439E | 100 | 170 |

(continued)

| Fc-GDF15 Construct | Fc mutation at position 356 or 439 | Expression volume (ml) | Expression yield (Protein A purification, μg/ml culture medium) |
|---|---|---|---|
| M4 | K439E | 100 | 169 |
| M5 | K439D | 100 | 137 |
| M6 | K439Q | 100 | 294 |
| M7 | K439A | 100 | 260 |
| M8 | E356K | 100 | 345 |
| M9 | E356R | 100 | 272 |
| M10 | E356Q | 100 | 150 |
| M11 | E356A | 100 | 132 |
| M39 | K439Q | 100 | 358 |
| M50 | E356Q, K439Q | 100 | 215 |
| M53 | K439E | ΔN4 | 8.3 |
| M54 | E356D | ΔN4 | 33.7 |
| M55 | K439R | ΔN4 | 36.7 |

**Example 4 Physicochemical properties of Fc-GDF15 fusion protein**

The physicochemical properties of protein molecules, such as their tendency to aggregate and degrade, affect the druggability of the molecule, so an ideal drug molecule needs to have a stable single component, that is, less aggregation and degradation occur.

In order to test the physicochemical properties of the Fc-GDF15 fusion protein, the Fc-GDF15 fusion protein was expressed and purified by the method described in Example 2, and then the fusion protein was analyzed by size exclusion chromatography (SEC). Specifically, a Waters Xbridge BEH 200A, 3.5 μm (7.8 × 300 mm) chromatographic column was used for SEC analysis, the mobile phase was 150 mM phosphate buffer/ acetonitrile (9: 1, pH 6.5), and the flow rate was 0.5 ml/min. In the test results, the proportion of high molecular weight (HMW) components represents the proportion of aggregation, and the proportion of low molecular weight (LMW) components represents the proportion of degradation. The test results are shown in Table 6.

Table 6 Comparison of physicochemical properties of different Fc-GDF15 fusion proteins

| Construct | Fc mutation at position 356 or 439 | GDF 15 mutation | HMW(%) | LMW(%) |
|---|---|---|---|---|
| M1 | Unmutated | ΔN4 | 50.2 | 0.5 |
| M14 | Unmutated | ΔN4 | 67.6 | 0.7 |
| M2 | K439E | WT | 3.6 | 2.1 |
| M3 | K439E | ΔN3 | 2.4 | 0.1 |
| M4 | K439E | ΔN4 | 2.2 | 0.7 |
| M5 | K439D | ΔN4 | 2.8 | 0.2 |
| M6 | K439Q | ΔN4 | 2.7 | 1.0 |
| M7 | K439A | ΔN4 | 4.4 | 0.9 |
| M8 | E356K | ΔN4 | 7.4 | 0.6 |
| M9 | E356R | ΔN4 | 3.9 | 0.3 |
| M10 | E356Q | ΔN4 | 7.1 | 0.7 |
| M11 | E356A | ΔN4 | 11.2 | 1.7 |

(continued)

| Construct | Fc mutation at position 356 or 439 | GDF 15 mutation | HMW(%) | LMW(%) |
|---|---|---|---|---|
| M13 | K439E | △N4 | 2.2 | 0.2 |
| M15 | K439E | △N4 | 2.1 | 0.2 |
| M16 | K439E | △N4 | 12 | 1.5 |
| M17 | K439E | △N4 | 11.0 | 0.3 |
| M18 | K439E | △N4 | 0.2 | 0.1 |
| M19 | K439E | △N4 | 4.6 | 0.7 |
| M20 | K439E | △N4, D5E | 2.2 | 0.1 |
| M21 | K439E | △N5, H6D | 2.0 | 1.1 |
| M22 | K439E | △N4, H6E | 2.1 | 0.2 |
| M23 | K439E | △N5, H6E | 1.8 | 0.2 |
| M24 | K439E | △N4, R21Q | 3.1 | 0.1 |
| M25 | K439E | △N4, R21H | 1.5 | 0.1 |
| M26 | K439E | △N4, D26E | 2.3 | 0.1 |
| M27 | K439E | △N4, D26E, K69R | 2.8 | 0.2 |
| M28 | K439E | △N4, A30S | 2.3 | 0.1 |
| M29 | K439E | △N4, A47D | 1.6 | 0.1 |
| M30 | K439E | △N4, A54S | 2.0 | 0.2 |
| M31 | K439E | △N4, A55E | 0.7 | 0.1 |
| M32 | K439E | △N4, M57T | 2.0 | 0.1 |
| M33 | K439E | △N4, R67Q | 1.6 | 0.2 |
| M34 | K439E | △N4, A81S | 2.3 | 0.1 |
| M35 | K439E | △N4, T94E | 0.7 | 0.1 |
| M36 | K439E | △N4, K107Q | 1.2 | 0.1 |
| M37 | K439E | △N14 | 1.4 | 1.1 |
| M39 | K439Q | △N3 | 3.2 | 0.9 |
| M50 | E356Q, K439Q | △N3 | 24.1 | 0.3 |
| M53 | K439E | △N4 | 8.3 | 2.7 |
| M54 | E356D | △N4 | 33.7 | 0.7 |
| M55 | K439R | △N4 | 36.7 | 0.6 |

The results in Table 6 show that Fc-GDF15 fusion proteins without mutating at K439 (EU numbering) or E356 (EU numbering) of Fc, such as M1 and M14, had 50.2% and 67.6% high molecular weight (HMW) aggregation, respectively; while Fc-GDF15 fusion proteins mutated at K439 (EU numbering) and/or E356 (EU numbering) of Fc all had significantly reduced polymer aggregation ratio, and most fusion proteins had less than 5% polymer aggregation, indicating that the mutation of Fc at K439 (EU numbering) or E356 (EU numbering) could bring better druggability of Fc-GDF15 fusion protein.

### Example 5 *In vitro* activity of Fc-GDF15 fusion protein

Previous studies have shown that the function of GDF15 *in vivo* requires signal transduction through its specific receptor GFRAL and coreceptor RET. GDF15 binding with its receptor on cell surface can activate downstream signaling pathways, one of which is ERK1/2 phosphorylation. Therefore, the *in vitro* activity and potency of GDF15 can be assessed by detecting the phosphorylation level of ERK1/2 in cells.

The gene sequences of human GFRAL (UniProtKB - Q6UXV0) and human RET (UniProtKB - P07949) were linked by IRES elements and placed downstream of the CMV promoter, and then transfected into HEK293T cells (ATCC). Puromycin (Gibco) was added to screen for positive cells stably expressing the two receptors, referred to as receptor-expressing cells for short, which were used for *in vitro* GDF15 activity analysis. The phosphorylation level of ERK1/2 in cells was detected using the Advanced phospho-ERK1/2 (Thr202/Tyr204) HTRF (Homogeneous Time-Resolved Fluorescence) kit (Cisbio), according to the manufacturer's instructions.

Briefly, the receptor-expressing cells were plated in a 384-well plate at a density of 16,000 cells/well. After starvation, the cells were stimulated with the Fc-GDF15 fusion proteins or natural GDF15 (ACROBiosystems, GD5-H5149). After stimulation, the cells were lysed and incubated with labeled antibody (supplied with the assay kit) overnight at room temperature. A multifunctional microplate reader (SpectraMax i3X, Molecular devices) was used to detect the fluorescence values at 665 nm and 620 nm with 337 nm as the excitation wavelength. The signal intensity is calculated by the ratio of the fluorescence values at 665nm and 620nm, and the formula is as follows:

$$\mathrm{Ratio} = \frac{A_{665}}{A_{620}} \times 10^4$$

Among them: A665 is the fluorescence value detected at 665nm wavelength; A620 is the fluorescence value detected at 620nm wavelength. The intensity of the response value corresponds to the phosphorylation intensity of ERK1/2 in cells.

$EC_{50}$ for each construct is determined using a variable slope sigmoidal dose-response curve with four parameter logistic regression in GraphPad Prism, and then compared with the activity $EC_{50}$ of natural mature GDF15. The relative activity ratio (native GDF15 $EC_{50}$: Fc-GDF15 construct $EC_{50}$) was calculated. The results are shown in Table 7.

Table 7 *In vitro* activity of Fc-GDF15 fusion protein

| Construct | mutation of Fc at position 356 or 439 | GDF15 mutation | Relative activity ratio |
|---|---|---|---|
| native GDF15 | N/A | WT | 1 |
| M1 | Unmutated | ΔN4 | 2.66 |
| M2 | K439E | WT | 3.27 |
| M3 | K439E | ΔN3 | 4.53 |
| M4 | K439E | ΔN4 | 2.62 |
| M5 | K439D | ΔN4 | 2.47 |
| M6 | K439Q | ΔN4 | 2.71 |
| M7 | K439A | ΔN4 | 3.16 |
| M8 | E356K | ΔN4 | 1.69 |
| M9 | E356R | ΔN4 | 1.33 |
| M10 | E356Q | ΔN4 | 1.62 |
| M11 | E356A | ΔN4 | 2.02 |
| M12 | K439E | ΔN4 | 0.97 |
| M13 | K439E | ΔN4 | 1.89 |
| M14 | Unmutated | ΔN4 | 1.18 |
| M15 | K439E | ΔN4 | 2.79 |
| M16 | K439E | ΔN4 | 2.03 |
| M17 | K439E | ΔN4 | 1.31 |
| M18 | K439E | ΔN4 | 1.69 |
| M19 | K439E | ΔN4 | 1.19 |
| M20 | K439E | ΔN4, D5E | 3.08 |

(continued)

| Construct | mutation of Fc at position 356 or 439 | GDF15 mutation | Relative activity ratio |
|---|---|---|---|
| M21 | K439E | ΔN5, H6D | 3.87 |
| M22 | K439E | ΔN4, H6E | 1.42 |
| M23 | K439E | ΔN5, H6E | 3.38 |
| M24 | K439E | ΔN4, R21Q | 2.65 |
| M25 | K439E | ΔN4, R21H | 4.76 |
| M26 | K439E | ΔN4, D26E | 2.91 |
| M27 | K439E | ΔN4, D26E, K69R | 2.66 |
| M28 | K439E | ΔN4, A30S | 1.38 |
| M29 | K439E | ΔN4, A47D | 1.26 |
| M30 | K439E | ΔN4, A54S | 1.51 |
| M31 | K439E | ΔN4, A55E | 0.87 |
| M32 | K439E | ΔN4, M57T | 1.26 |
| M33 | K439E | ΔN4, R67Q | 0.84 |
| M34 | K439E | ΔN4, A81S | 0.90 |
| M35 | K439E | ΔN4, T94E | 0.19 |
| M36 | K439E | ΔN4, K107Q | 0.82 |
| M37 | K439E | ΔN14 | 3.17 |
| M39 | K439Q | ΔN3 | 2.62 |
| M50 | E356Q, K439Q | ΔN3 | 1.50 |
| M51 | K439N | ΔN4 | 3.16 |

The results show that except for M35, which had significantly reduced *in vitro* activity, the remaining Fc-GDF15 fusion proteins all had *in vitro* activities comparable to or better than native GDF15.

**Example 6 Thermal stability of Fc-GDF15 fusion protein (DSC)**

In this example, differential scanning calorimetry (DSC) was used to test the thermal stability of different Fc-GDF15 fusion proteins. The endothermic transition curve on the DSC spectrum represents the denaturation and unfolding process of the protein molecule, and the thermal stability of the protein can be characterized by the thermal transition midpoint (Tm) value. Specifically, the samples were heated from 30°C to 110°C at a heating rate of 120°C/hour, and the Tm values of different Fc-GDF15 fusion proteins were determined. The results are shown in Table 8. The experimental results show that the Fc-GDF15 fusion proteins have good thermal stability and are suitable for drug development.

Table 8 Tm value of Fc-GDF15 fusion protein

| Construct | Tm (°C) |
|---|---|
| M3 | 63.94 |
| M4 | 63.93 |
| M5 | 62.74 |
| M6 | 65.69 |
| M15 | 65.23 |
| M39 | 65.71 |

**Example 7 *In vivo* efficacy of single-dose administration of Fc-GDF15 fusion protein in normal mice**

GDF15 can inhibit food intake, reduce body weight, and has the potential to treat obesity and other related metabolic diseases. However, the natural GDF15 molecule has only a serum half-life of about 3 hours, which largely limited its direct therapeutic application. The Fc-GDF15 fusion protein provided by the present invention can prolong the half-life of the drug molecule. In this example, the effect of single-dose administration of different Fc-GDF15 fusion proteins on food intake and body weight of normal mice were tested to evaluate the *in vivo* efficacy and duration of action of different Fc-GDF15 fusion proteins.

Specifically, 8-9 week old C57BL/6 mice (Hunan SJA Laboratory Animal) were weighed on day 0 and administered single subcutaneous doses of the Fc-GDF15 fusion proteins to be tested or vehicle (PBS), and the food intake and body weight were continuously monitored after administration. The calculation method of weight change percentage is: weight change (%) = (measured weight - initial weight)/initial weight $\times$ 100%

The results of food intake and body weight of normal mice treated with a single dose for 14 days are shown in Table 9, Figure 2 and Figure 3.

Table 9 Effect of single-dose treatment on body weight and food intake in normal mice (monitored for 14 days)

| Group | Day 8 | | Day 14 | |
|---|---|---|---|---|
| | Weight change (%) | Cumulative food intake (g) | Weight change (%) | Cumulative food intake (g) |
| vehicle | 5.5$\pm$1.1 | 35.1 | 6.3$\pm$1.3 | 61.9 |
| MonoFc-GDF15 (3nmol/kg) | -5.7$\pm$1.4[***] | 28.1 | -7.1$\pm$2.5[***] | 50.4 |
| M3 (3nmol/kg) | -8.4$\pm$1.8[***] | 28.7 | -15.4$\pm$2.2[***#] | 48.9 |
| M15 (3nmol/kg) | -8.4$\pm$1.8[***] | 27.9 | -14.5$\pm$2.0[***#] | 48.0 |
| M20 (3nmol/kg) | -10.8$\pm$1.0[***#] | 23.7 | -16.2$\pm$0.8[***##] | 42.2 |
| M23 (3nmol/kg) | -11.9$\pm$2.0[***#] | 24.2 | -16.1$\pm$2.4[***#] | 43.6 |
| M24 (3nmol/kg) | -10.1$\pm$0.9[***#] | 25.0 | -13.8$\pm$1.6[***#] | 44.1 |
| M35 (3nmol/kg) | -6.6$\pm$1.2[***] | 27.4 | -13.7$\pm$1.5[***#] | 48.5 |
| Note: All data are presented as mean $\pm$ SEM of 7 animals per group; ***-p<0.001, vs. vehicle; #-p<0.05, vs. MonoFc-GDF15; ##-p<0.01, vs. MonoFc-GDF15, statistical analysis method: T-test. | | | | |

The results show that a single administration of the Fc-GDF15 fusion proteins at a dose of 3 nmol/kg in normal mice suppressed food intake and significantly decreased body weight relative to vehicle on the 8th day and the 14th day. Among them, the GDF15 fusion protein monoFc-GDF15 containing monomeric Fc ("Compound 2" disclosed in patent WO2019195091, the monomer has the sequence of SEQ ID NO: 104; Fc has the sequence of SEQ ID NO: 18, wherein F405Q, Y407E) reached the optimal efficacy on the 8th day; Fc-GDF15 fusion proteins mutated at K439 of Fc, such as M3 (a homodimer with a monomer having the sequence of SEQ ID NO: 68) and M15 (a homodimer with a monomer having the sequence of SEQ ID NO: 80); and Fc-GDF15 fusion proteins further comprising mutations at different amino acid sites of GDF15, such as M20 (a homodimer with a monomer having the sequence of SEQ ID NO: 85), M23 (a homodimer with a monomer having the sequence of SEQ ID NO: 88), M24 (a homodimer with a monomer having the sequence of SEQ ID NO: 89) and M35 (a homodimer with a monomer having the sequence of SEQ ID NO: 100) still showed a downward trend in the body weight of mice on the 14th day after administration, and the weight loss was significantly greater than the effect of the monoFc-GDF15 fusion protein.

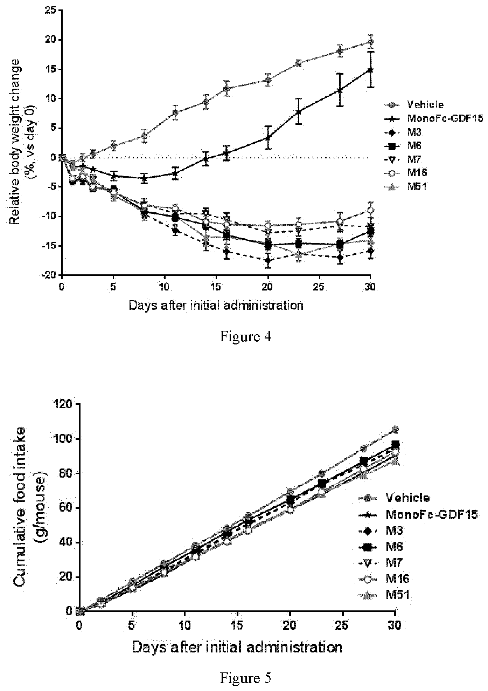

The results of food intake and body weight of normal mice treated with a single dose for 30 days are shown in Table 10, Figure 4 and Figure 5.

Table 10 Effect of single-dose treatment on body weight and food intake in normal mice (monitored for 30 days)

| Group | Day 8 | | Day 14 | |
|---|---|---|---|---|
| | Weight change (%) | Cumulative food intake (g) | Weight change (%) | Cumulative food intake (g) |
| vehicle | 3.2±2.8 | 27.7 | 7.9±5.2 | 48.4 |
| MonoFc-GDF15 (1nmol/kg) | -3.6±2.2*** | 21.5 | -0.2±3.2** | 41.3 |
| M3 (1nmol/kg) | -9.6±1.8***### | 23.7 | -14.6±3.5***### | 44.3 |
| M6 (1nmol/kg) | -9.1±2.5***### | 26.0 | -11.4±2.1***### | 46.2 |
| M7 (1nmol/kg) | -8.0±2.8***## | 22.8 | -9.6±2.8***### | 43.7 |
| M16 (1nmol/kg) | -8.2±2.2***## | 22.8 | -10.9±2.4***### | 40.5 |
| M51 (1nmol/kg) | -9.2±3.9***## | 22.5 | -13.6±4.2***### | 41.3 |
| Note: All data are presented as mean ± SEM of 7-8 animals per group; **-p<0.01, ***-p<0.001, vs. vehicle; ##-p<0.01, ###-p<0.001, vs. MonoFc-GDF15, statistical analysis method: T-test. | | | | |

The results show that a single administration of the Fc-GDF15 fusion proteins at a dose of 1nmol/kg in normal mice could significantly reduce the body weight of the mice. Among them, the GDF15 fusion protein MonoFc-GDF15 containing monomeric Fc had a weaker weight-reducing effect, and the mice had recovered to their initial body weight on the 14th day. However, Fc-GDF15 constructs containing different amino acid mutations of Fc at position K439, such as M3 (a homodimer with a monomer having the sequence of SEQ ID NO: 68), M6 (a homodimer with a monomer having the sequence of SEQ ID NO: 71), M7 (a homodimer with a monomer having the sequence of SEQ ID NO: 72), M16 (a homodimer with a monomer having the sequence of SEQ ID NO: 81) and M51 (a homodimer with a monomer having the sequence of SEQ ID NO: 117), had significantly stronger weight-reducing effect, and could continuously reduce the weight of mice till up to 21-28 days, suggesting a significantly prolonged drug effect maintenance time.

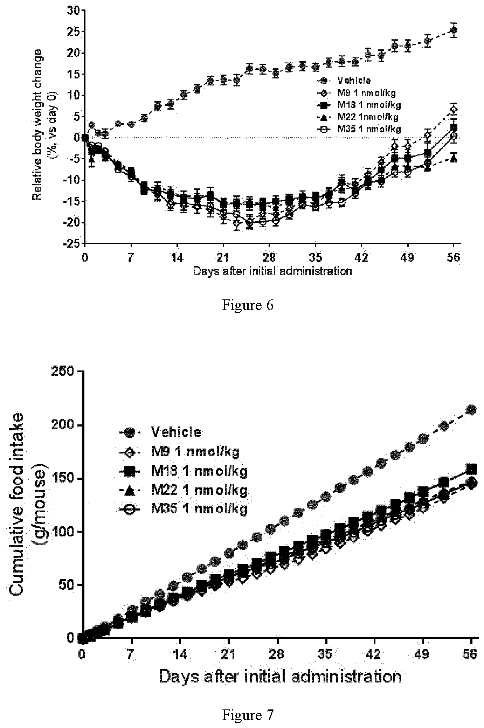

The results of food intake and body weight of normal mice treated with a single dose for 56 days are shown in Table 11, Figure 6 and Figure 7.

Table 11 Effect of single-dose treatment on body weight and food intake in normal mice (monitored for 56 days)

| Group | Day 13 | | Day 25 | | Day 41 | |
|---|---|---|---|---|---|---|
| | Weight change (%) | Cumulative food intake (g) | Weight change (%) | Cumulative food intake (g) | Weight change (%) | Cumulative food intake (g) |
| vehicle | 7.9±1.1 | 49.4 | 16.3±1.2 | 95.2 | 17.9±1.0 | 156.6 |
| M9 (1nmol/kg) | -15.1±1.5*** | 35.2 | -19.6±1.5*** | 60.6 | -11.0±1.3*** | 100.9 |
| M18 (1nmol/kg) | -13.3±1.1*** | 38.4 | -15.7±1.3*** | 70.7 | -12.4±1.9*** | 114.4 |
| M22 (1nmol/kg) | -12.7±1.2*** | 37.5 | -14.9±1.0*** | 67.8 | -12.1±0.8*** | 108.3 |
| M35 (1nmol/kg) | -15.7±1.4*** | 35.7 | -20.0±1.4*** | 66.6 | -13.1±1.2*** | 105.5 |
| Note: All data are presented as mean ± SEM of 8 animals per group; ***-p<0.001, vs. vehicle; statistical analysis method: T-test. | | | | | | |

The results show that single dose treatment of 1 nmol/kg of M9 (a homodimer with a monomer having the sequence of SEQ ID NO: 74), M18 (a homodimer with a monomer having the sequence of SEQ ID NO: 83), M22 (a homodimer with a monomer having the sequence of SEQ ID NO: 87) or M35 (a homodimer with a monomer having the sequence of SEQ ID NO: 100) could inhibit food intake and significantly reduce weight in normal mice. Moreover, the body weight of the mice continued to decrease till around day 21 before rebound, and returned to baseline on around day 56, indicating that Fc-GDF15 fusion proteins mutated at Fc K439 or E356, such as M9 and M18, as well as fusion proteins that further contained mutations at different amino acid sites of GDF15, such as M22 and M35, all had super-long duration of efficacy. The body weight lowering efficacy could be maintained for about 1 month after a single-dose of administration. At the same time, the inventors surprisingly found that although construct M35 had significantly reduced *in vitro* activity, it still maintained similar *in vivo* efficacy as other constructs.

**Example 8 *In vivo* efficacy of repeated-dose administration of Fc-GDF15 fusion proteins in diet-induced obesity (DIO) mice**

This example tests the effect of repeated-dose administration of Fc-GDF15 fusion proteins on appetite suppression, body weight reduction, and improvement of various metabolic indicators in DIO mice. After four months of induction with high-fat diet, C57BL/6 mice were randomly grouped according to body weight, and then subcutaneously administered with vehicle (PBS, once every two weeks), different Fc-GDF15 fusion proteins (1nmol/kg, once every two weeks) or Semaglutide (3nmol/kg, once a day) for 49 days. Changes in body weight and food intake of mice were continuously monitored during the experiment. Intraperitoneal glucose tolerance tests (IPGTT) were performed on the 45th day after the initial administration, and on the 49th day, the mice were sacrificed and the fat content and various serum biochemical parameters were analyzed.

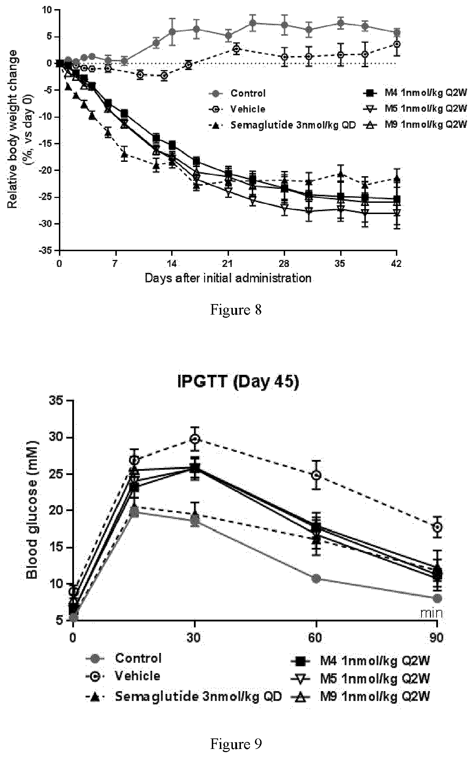

The results of body weight change and food intake of mice are shown in Table 12, the body weight change curve is shown in Figure 8; the IPGTT data is shown in Figure 9; the parameters of area under the glucose tolerance curve, fat index, serum total cholesterol (TC), triglycerides (TG), alanine transaminase (ALT), aspartate transaminase (AST) and low density lipoprotein (LDL) are shown in Table 13.

Table 12 Effect of repeated administration of Fc-GDF15 constructs on body weight and food intake in DIO mice (day 42)

| Group | Weight change (%) | Cumulative food intake (g/mouse) |
|---|---|---|
| vehicle | 3.7±2.2 | 111.4 |
| Semaglutide (3nmol/kg) | -21.4±1.7 | 92.7 |
| M4 (1nmol/kg) | -25.3±3.1 | 93.8 |
| M5 (1nmol/kg) | -28.0±2.9 | 93.8 |
| M9 (1nmol/kg) | -25.9±4.3 | 92.1 |
| [00151]Note: All data are represented as mean ± SEM of 9 animals in each group. | | |

Table 13 Effect of repeated administration of Fc-GDF15 constructs on glucose tolerance, fat index, serum TC, TG, ALT, AST and LDL in DIO mice

| Group | IPGTT blood glucose $AUC_{0-90min}$ | Fat index (%) | TC(mmol /L) | TG(mmol /L) | ALT(U/L) | AST(U/L) | LDL(mmol /L) |
|---|---|---|---|---|---|---|---|
| control | 1198.6±23. 6*** | 2.57±0.41*** | 2.31±0.23 *** | 0.77±0.04 *** | 29.42±1.67*** | 112.67±6.39*** | 0.36±0.02* ** |
| vehicle | 2257.6±136 .1 | 14.24±1. 06 | 4.35±0.18 | 1.73±0.13 | 82.41±11. 97 | 250.00±20. 02 | 0.77±0.07 |
| Semaglut ide (3nmol/k g) | 1450.5±144 .9** | 7.62±1.3 0** | 3.24±0.21 * | 0.80±0.08 *** | 23.79±1.47*** | 115.44±4.6 3 *** | 0.42±0.04* * |
| M4 (1nmol/k g) | 1679.3±90. 4* | 6.35±0.85*** | 3.17±0.28 * | 0.97±0.07 *** | 31.32±5.95*** | 116.22±6.34*** | 0.41±0.06* * |
| M5 (1nmol/k g) | 1654.4±116 .6* | 5.75±1.23*** | 3.21±0.12 * | 1.00±0.10 *** | 27.94±2.69*** | 106.00±3.34*** | 0.39±0.03* ** |
| M9 (1nmol/k g) | 1750.4±113 .1ns | 6.37±1.12*** | 3.34±0.20 * | 0.93±0.05 *** | 30.17±4.23*** | 119.11±7.71*** | 0.43±0.05* * |

Note: All data are represented as mean ± SEM of 7-9 animals per group; *-p<0.05, **-p<0.01, ***-p<0.001, ns-no significant difference, vs. vehicle; statistical analysis method: one-way ANOVA, followed by Dunnett's.

The results show that the administration of Fc-GDF15 fusion proteins M4 (a homodimer with a monomer having the sequence of SEQ ID NO: 69), M5 (a homodimer with a monomer having the sequence of SEQ ID NO: 70) or M9 (a homodimer with a monomer having the sequence of SEQ ID NO: 74) at a dose of 1 nmol/kg every two weeks could inhibit food intake and significantly reduce the body weight of DIO mice with the efficacy comparable to that of 3 nmol/kg Semaglutide daily administration, which could make the weight of the mice reach a normal level; M4 and M5 could also significantly improve the glucose tolerance level of DIO mice; M4, M5 and M9 could significantly reduce the fat content and blood lipids including total cholesterol, triglycerides and low density lipoprotein; M4, M5 and M9 could also improve liver function such as significantly reducing ALT and AST levels. It is shown that the Fc-GDF15 construct of the present invention can be applied to the treatment of obesity, diabetes, hyperlipidemia or fatty liver/steatohepatitis.

In another experiment, the effect of repeated-dose administration of different doses of construct M39 (a homodimer with a monomer having the sequence of SEQ ID NO: 105) on suppressing appetite, reducing body weight, and improving various metabolic parameters in DIO mice was tested. DIO mice were randomly divided into groups (10-12 mice per group) and were subcutaneously administered with vehicle (PBS, once a week), different doses of M39 (0.03nmol/kg, 0.1nmol/kg and 1nmol/kg, once a week; 3nmol/kg, once every two weeks) or Semaglutide (3nmol/kg, once a day). The changes in body weight and food intake of the mice were continuously monitored. Fasting blood glucose and glucose tolerance were measured on day 46 after the initial administration, and the mice were sacrificed on day 49, and the fat content and various serum biochemical indicators were analyzed.

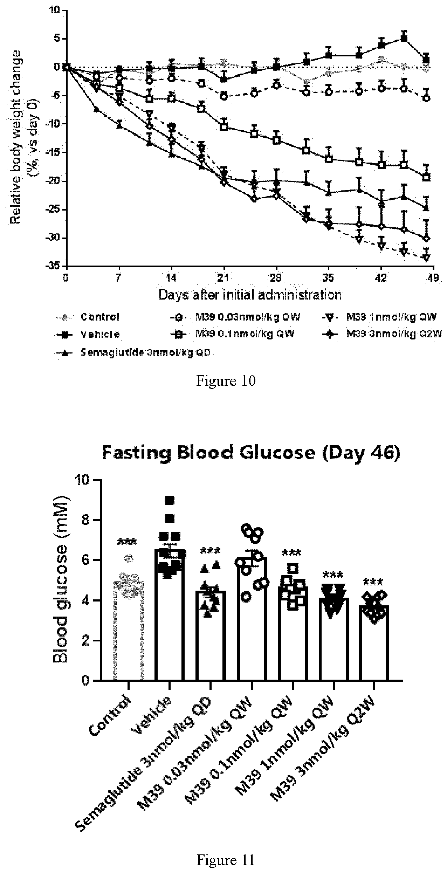

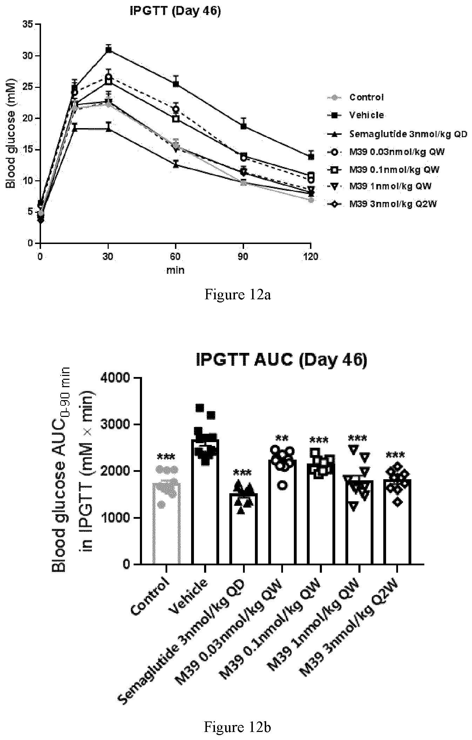

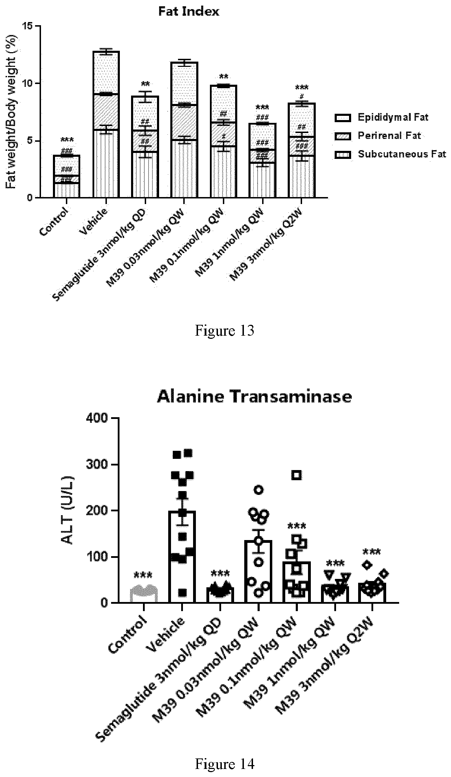

The body weight change of mice at the end of treatment is shown in Table 14, and the body weight change curve, fasting blood glucose level, IPGTT (on day 46) and area under the curve, fat index, and transaminase levels are shown in Figures 10 to 15, respectively.

Table 14 Effect of repeated administration of different doses of M39 on weight loss in DIO mice (day 48)

| Group | Weight change (% from baseline) |
|---|---|
| Vehicle | 1.2±1.1 |
| Semaglutide QD (3nmol/kg) | -24.7±2.0*** |
| M39 QW (0.03nmol/kg) | -5.4±1.6 |
| M39 QW (0.1nmol/kg) | -19.4±2.2*** |
| M39 QW (1nmol/kg) | -33.6±1.8***# |
| M39 Q2W (3nmol/kg) | -30.1±3.2*** |
| Note: All data are presented as mean ± SEM of 10-12 animals per group; ***-p<0.001 vs. vehicle, #-p<0.05 vs. Semaglutide; Statistical analysis method: one-way ANOVA, followed by Dunnett's. | |

The above results show that subcutaneous injection of M39 once a week or once every two weeks could reduce the body weight of DIO mice in a dose-dependent manner, and the weight loss effect of 1 nmol/kg once a week was significantly stronger than that of 3 nmol/kg Semaglutide daily administration. At the same time, M39 could also significantly reduce the fat content of mice, improve the fasting blood glucose level, glucose tolerance, and liver function biomarkers in mice.

**Example 9 *In vivo* efficacy of Fc-GDF15 fusion proteins in ob/ob obese mouse model**

This example tests the effect of constructs M6 and M39 on suppressing appetite, reducing body weight, and improving various metabolic indicators in ob/ob obese mouse model. After acclimation, ob/ob mice of 6-7 weeks old (GemPharmatech) were randomly divided into groups according to body weight, and were subcutaneously administered with vehicle (PBS), different doses of M6 or M39 (0.1 nmol/kg, dosed on day 0 and day 24; 1 nmol/kg, 10 nmol/kg, dosed on day 0 and day 36) or Semaglutide (3 nmol/kg, once a day). Changes in body weight and food intake of the mice were continuously monitored. Mice were sacrificed on day 52, and various serum biochemical indicators and liver pathology were analyzed.

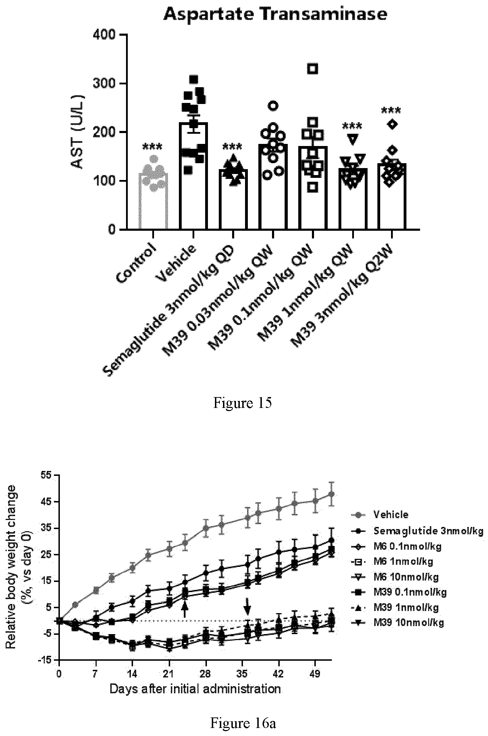

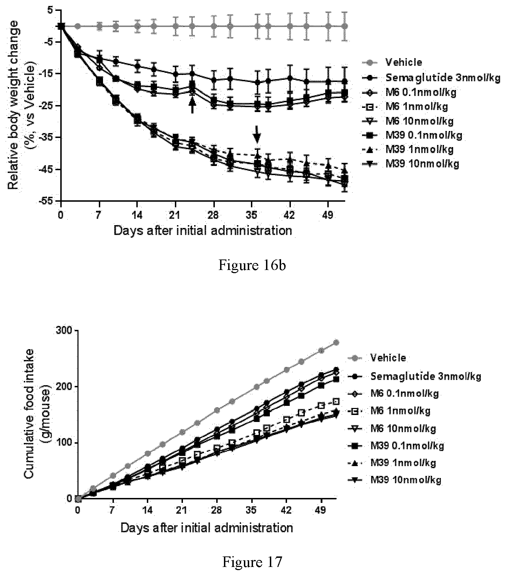

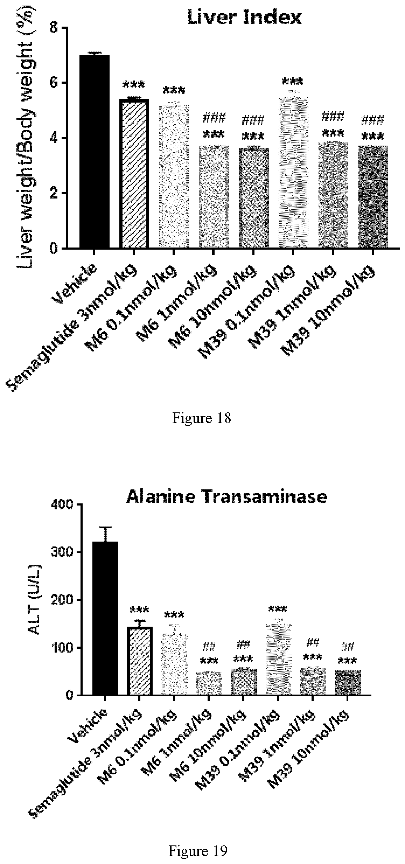

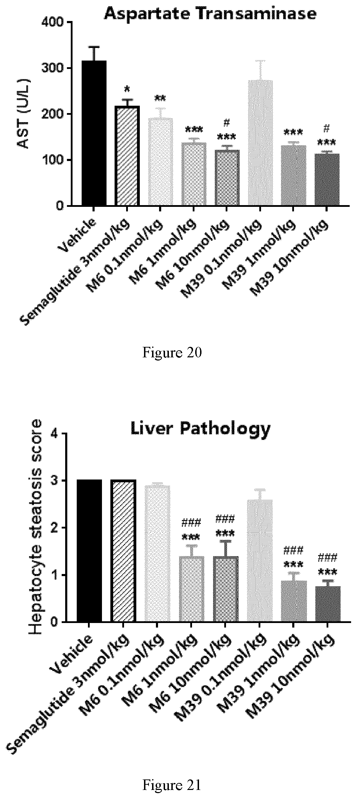

The body weight changes of mice at the end of treatment are shown in Table 15, and the results of body weight change curve, cumulative food intake, liver index, transaminase levels, and liver pathology are shown in Figures 16 to 21, respectively.

Table 15 Effect of different doses of M6 and M39 on improving the body weight of ob/ob mice (day 52)

| Group | Weight change (% from baseline) |
|---|---|
| Vehicle | 47.9±4.5 |
| Semaglutide QD (3nmol/kg) | 30.6±4.5*** |
| M6 (0.1nmol/kg) | 25.9±1.7*** |
| M6 (1nmol/kg) | 0.1±2.0***### |
| M6 (10nmol/kg) | -1.9±2.2***### |
| M39 (0.1nmol/kg) | 27.2±2.8*** |
| M39 (1nmol/kg) | 2.7±2.1***### |
| M39 (10nmol/kg) | -0.7±1.7***### |
| Note: All data are presented as mean ± SEM of 8 animals per group; ***-p<0.001 vs. vehicle, ###-p<0.001 vs. Semaglutide; Statistical analysis method: one-way ANOVA, followed by Dunnett's. | |

The above results show that the Fc-GDF15 constructs designed by the present invention had an ultra-long duration of drug efficacy. M6 and M39 administered once on the 0 day and the 24th day at low doses and once on the 0 day and the 36th day at medium and high doses could significantly reduce food intake in ob/ob mice. The improvement of body weight was significantly stronger than that of Semaglutide administered daily, and it could also significantly improve liver function and hepatic steatosis in ob/ob mice, and the effect was also significantly better than that of Semaglutide.

**Example 10 Pharmacokinetics of Fc-GDF15 fusion proteins in mice**

This example tested the pharmacokinetic performance of constructs M38 (a homodimer with a monomer having the sequence of SEQ ID NO: 103), M3 (a homodimer with a monomer having the sequence of SEQ ID NO: 68), M15 (a homodimer with a monomer having the sequence of SEQ ID NO: 80) in mice. The above fusion proteins were diluted to 0.05 mg/ml with 10 mM PBS, and then C57BL/6 mice were subcutaneously injected at a dose of 0.25 mg/kg, with 3 mice per dose group. Blood was collected from each animal before administration, and 2h, 7h, 24h, 48h, 96h, 168h, 356h, 504h, and 672h after administration, respectively, and the plasma Fc-GDF15 concentrations were determined by sandwich ELISA. Specifically, mouse anti-human GDF15 monoclonal antibody (Sino Biological Inc.) was used to coat the plate, mouse anti-human IgG4 Fc-HRP was used to detect the target protein, and then the pharmacokinetic data was calculated. The results are shown in Table 16.

Table 16 Pharmacokinetic parameters of Fc-GDF15 fusion proteins in mice

| Construct | PK parameter | | | | | | | |
|---|---|---|---|---|---|---|---|---|
| | $AUC_{INF\_obs}$ (h*ng/ml) | | $C_{max}$ (ng/ml) | | $T_{1/2}$ (h) | | $T_{max}$ (h) | |
| | Average value | SD (standard deviation) | Average value | SD (standard deviation) | Average value | SD (standard deviation) | Average value | SD (standard deviation) |
| M38 | 42100 | 3900 | 541 | 46 | 58 | 2.9 | 7 | 0 |
| M15 | 314000 | 17000 | 904 | 55 | 244 | 13 | 30 | 12 |
| M3 | 397000 | 55000 | 907 | 95 | 306 | 24 | 48 | 34 |
| Note: $AUC_{INF\_obs}$ represents the area under the curve from the time of administration to the theoretical extrapolation infinity; $C_{max}$ represents the highest detected plasma concentration; $T_{1/2}$ represents the half-life; $T_{max}$ represents the time to reach the highest plasma concentration. | | | | | | | | |

The results show that the fusion protein M38 containing monomeric Fc and GDF15 had a half-life of 58h in mice; surprisingly, it was found that the half-lives of Fc-GDF15 fusion proteins M15 and M3 with Fc variants in mice reached 244h and 306h, much longer than the half-lives of conventional Fc fusion proteins in mice.

**Example 11 Pharmacokinetics of Fc-GDF15 fusion protein in cynomolgus monkeys**

This example tested the pharmacokinetic parameters of construct M39 in cynomolgus monkeys. M39 was administered subcutaneously to cynomolgus monkeys at doses of 1 mg/kg and 2 mg/kg, respectively, in three animals of each sex. Blood was collected from each animal before administration, and 0.5h, 2h, 6h, 48h, 168h, 356h, 504h, and 672h after administration, and the plasma Fc-GDF15 concentration was determined by sandwich ELISA. Specifically, mouse anti-human GDF15 monoclonal antibody (Sino Biological Inc.) was used to coat the plate, mouse anti-human IgG4 Fc-HRP was used to detect the target protein, and then the pharmacokinetic data was calculated. The results are shown in Table 17.

Table 17 Pharmacokinetic parameters of construct M39 in cynomolgus monkeys

| | | PK parameter | | | | | | | |
|---|---|---|---|---|---|---|---|---|---|
| | | $AUC_{INF\_obs}$ (h*ug/ml) | | $C_{max}$ (ug/ml) | | $T_{1/2}$ (h) | | $T_{max}$ (h) | |
| Dose | Sex | Mean | SD (standard deviation) | Mean | SD (standard deviation ) | Mean | SD (standard deviation) | Mean | SD (standard deviation ) |
| 1mg/kg | female | 7480 | 1740 | 16 | 0.307 | 290 | 12 | 48 | 0 |
| 1mg/kg | male | 4950 | 282 | 10.7 | 1.49 | 260 | 7 | 34 | 24 |
| 2mg/kg | female | 13400 | 3640 | 31.2 | 4.53 | 240 | 42 | 48 | 0 |
| 2mg/kg | male | 9320 | 2040 | 26.9 | 4.96 | 210 | 55 | 34 | 24 |

The inventors were surprised to find that the construct M39 also showed an ultra-long half-life in cynomolgus monkeys, reaching 200-300 hours, much longer than the half-life of conventional non-antibody Fc fusion proteins in cynomolgus monkeys, which was about 100 hours.

The above description is only a preferred embodiment of the present invention, but the protection scope of the present invention is not limited to this. Any changes or substitutions that can be easily conceived by any person skilled in the art within the technical scope disclosed by the present invention shall be included within the protection scope of the present invention. Therefore, the protection scope of the present invention should be based on the protection scope of the claims.

**Claims**

1. AFc-GDF15 fusion protein for use in treating obesity-related diseases.

2. The fusion protein of claim 1, wherein the fusion protein comprises a GDF15 active domain and an Fc variant; the C-terminal of the Fc variant is linked directly or via a peptide linker to the N-terminal of the GDF15 active domain; the Fc variant comprises amino acid substitutions at position 356 and/or position 439 of the IgG Fc according to EU numbering.

3. The fusion protein of claim 2, wherein the Fc variant has the ability to form homodimers.

4. The fusion protein of any one of claims 2 to 3, wherein the Fc variant comprises an amino acid substitution at position 356 of the IgG Fc with amino acids other than aspartic acid (D), glutamic acid (E) and cysteine (C) according to EU numbering; and/or the Fc variant comprises an amino acid substitution at position 439 of the IgG Fc with amino acids other than arginine (R), histidine (H), lysine (K) and cysteine (C) according to EU numbering;

preferably, the Fc variant comprises an amino acid substitution at position 356 of the IgG Fc according to EU numbering with one of: glycine (G), serine (S), alanine (A), threonine (T), valine (V), asparagine (N), leucine (L), isoleucine (I), glutamine (Q), tyrosine (Y), phenylalanine (F), histidine (H), proline (P), methionine (M), lysine (K) and arginine (R), and/or the Fc variant comprises an amino acid substitution at position 439 of IgG Fc according to EU numbering with one of: glycine (G), serine (S), alanine (A), threonine (T), valine (V), aspartic acid (D), asparagine (N), leucine (L), isoleucine (I), glutamic acid (E), glutamine (Q), tyrosine (Y), phenylalanine (F), proline (P) and methionine (M);
more preferably, the Fc variant comprises the following mutations: one of E356R, E356Q, E356A, E356N, and/or one of K439D, K439E, K439Q, K439A, K439N.

5. The fusion protein of any one of claims 2 to 3, wherein the Fc variant comprises one of the following mutations: K439D, K439E, K439Q, K439A, K439N;
preferably, the Fc variant comprises one of the following mutations: K439D, K439E, K439Q.

6. The fusion protein of any one of claims 2 to 3, wherein the Fc variant comprises one of the following mutations: E356R, E356Q, E356A, E356N;
preferably, the Fc variant comprises the E356R mutation.

7. The fusion protein of any one of claims 1 to 6, wherein the Fc variant further comprises the following mutations: amino acid substitutions at position 234 and position 235 of the IgG Fc with alanine (AA), and/or amino acid deletion at position 447 of the IgG Fc according to EU numbering.

8. The fusion protein of any one of claims 1 to 7, wherein the Fc variant comprises an amino acid sequence of one of the following group, or an amino acid sequence having at least 85%, 90%, 95% or 99% sequence identity to one of the following group: SEQ ID NO: 1-22, SEQ ID NO: 27, SEQ ID NO: 29-45.

9. The fusion protein of any one of claims 2 to 3, wherein the GDF15 active domain is a full-length mature GDF15 protein, an N-terminal truncated GDF15 protein or any variant that retains the biological activity of GDF15.

10. The fusion protein of claim 9, wherein the GDF15 active domain comprises an amino acid sequence selected from one of the following group, or having at least 85%, 90%, 95% or 99% sequence identity to one of the following group: SEQ ID NO: 46;
1-14 amino acid truncations at the N-terminal of SEQ ID NO:46, and/or 1-3 amino acid substitutions in SEQ ID NO:46.

11. The fusion protein of claim 10, wherein the GDF15 active domain comprises an amino acid sequence selected from one of the following group:
SEQ ID NO: 46;
1-14 amino acid truncations at the N-terminal of SEQ ID NO:46, and/or 1-3 amino acid substitutions in SEQ ID NO:46.

12. The fusion protein of any one of claims 10 to 11, wherein the position of amino acid substitution in SEQ ID NO:46 is selected from one, two or three of the following group: position 5, position 6, position 21, position 26, position 30, position 47, position 54, position 55, position 57, position 67, position 69, position 81, position 94, position 107;
preferably, the amino acid substitution in the SEQ ID NO: 46 is selected from one, any two of the different positions, or any three of the different positions: D5E, H6D, H6E, R21Q, R21H, D26E, A30S, A47D, A54S, A55E, M57T, R67Q, K69R, A81S, T94E, K107Q.

13. The fusion protein of any one of claims 10 to 11, wherein the N-terminal of SEQ ID NO: 46 is truncated by 3, 4 or 14 amino acids.

14. The fusion protein of any one of claims 9 to 13, wherein the GDF15 active domain comprises an amino acid sequence of one of the following group, or an amino acid sequence having at least 85%, 90%, 95% or 99% sequence identity to one of the following group: SEQ ID NO: 46-66.

15. The fusion protein of claim 14, wherein the GDF15 active domain comprises an amino acid sequence of one of the following group, or an amino acid sequence having at least 95% sequence identity to one of the following group: SEQ ID NO: 46-66;
preferably, the GDF15 active domain comprises an amino acid sequence of one of the following group: SEQ ID NO: 46-66.

**16.** The fusion protein of any one of claims 1 to 15, wherein the fusion protein comprises an amino acid sequence of one of the following group, or an amino acid sequence having at least 85%, 90%, 95% or 99% sequence identity to one of the following group: SEQ ID NO: 67-78, SEQ ID NO: 80-102, SEQ ID NO: 105-118.

**17.** The fusion protein of claims 16, wherein the fusion protein comprises an amino acid sequence of one of the following group, or an amino acid sequence having at least 95% sequence identity to one of the following group: SEQ ID NO: 67-78, SEQ ID NO: 80-102, SEQ ID NO: 105-118;
preferably, the fusion protein comprises an amino acid sequence of one of the following group: SEQ ID NO: 67-78, SEQ ID NO: 80-102, SEQ ID NO: 105-118.

**18.** A homodimeric fusion protein comprising the fusion protein of any one of claims 1 to 17.

**19.** A biological material, wherein the biological material is any one of the following (i), (ii), (iii):

(i) a nucleic acid comprising a nucleotide sequence encoding the fusion protein of any one of claims 1 to 16;
(ii) a vector comprising the nucleic acid of (i);
(iii) a host cell containing the nucleic acid of (i) and/or the vector of (ii).

**20.** A pharmaceutical composition comprising the homodimeric fusion protein of claim 17 as an active ingredient, wherein the homodimeric fusion protein is present in the pharmaceutical composition in a therapeutically effective amount;
preferably, the pharmaceutical composition further comprises a pharmaceutically acceptable carrier.

**21.** A method of treating obesity-related diseases comprising administering to a subject a therapeutically effective amount of Fc-GDF15 fusion protein.

**22.** The method of claim 21, wherein the fusion protein comprises a GDF15 active domain and an Fc variant; the C-terminal of the Fc variant is linked directly or via a peptide linker to the N-terminal of the GDF15 active domain; the Fc variant comprises amino acid substitutions at position 356 and/or position 439 of the IgG Fc according to EU numbering.

**23.** The method of claim 22, wherein the Fc variant has the ability to form homodimers.

**24.** The method of claim 22, wherein the Fc variant comprises an amino acid substitution at position 356 of the IgG Fc with amino acids other than aspartic acid (D), glutamic acid (E) and cysteine (C) according to EU numbering; and/or the Fc variant comprises an amino acid substitution at position 439 of the IgG Fc with amino acids other than arginine (R), histidine (H), lysine (K) and cysteine (C) according to EU numbering;

preferably, the Fc variant comprises an amino acid substitution at position 356 of the IgG Fc according to EU numbering with one of: glycine (G), serine (S), alanine (A), threonine (T), valine (V), asparagine (N), leucine (L), isoleucine (I), glutamine (Q), tyrosine (Y), phenylalanine (F), histidine (H), proline (P), methionine (M), lysine (K) and arginine (R), and/or the Fc variant comprises an amino acid substitution at position 439 of IgG Fc according to EU numbering with one of: glycine (G), serine (S), alanine (A), threonine (T), valine (V), aspartic acid (D), asparagine (N), leucine (L), isoleucine (I), glutamic acid (E), glutamine (Q), tyrosine (Y), phenylalanine (F), proline (P) and methionine (M);
more preferably, the Fc variant comprises the following mutations: one of E356R, E356Q, E356A, E356N, and/or one of K439D, K439E, K439Q, K439A, K439N.

**25.** The method of claim 24, wherein the Fc variant comprises one of the following mutations: K439D, K439E, K439Q, K439A, K439N;
preferably, the Fc variant comprises one of the following mutations: K439D, K439E, K439Q.

**26.** The method of claim 24, wherein the Fc variant comprises one of the following mutations: E356R, E356Q, E356A, E356N;
preferably, the Fc variant comprises the E356R mutation.

**27.** The method of any one of claims 21-26, wherein the Fc variant further comprises the following mutations: amino acid substitutions at position 234 and position 235 of the IgG Fc with alanine (AA), and/or amino acid deletion at position 447 of the IgG Fc according to EU numbering.

28. The method of claim 27, wherein the Fc variant comprises an amino acid sequence of one of the following group, or an amino acid sequence having at least 85%, 90%, 95% or 99% sequence identity to one of the following group: SEQ ID NO: 1-22, SEQ ID NO: 27, SEQ ID NO: 29-45.

29. The method of claim 22, wherein the GDF15 active domain is a full-length mature GDF15 protein, an N-terminal truncated GDF15 protein or any variant that retains the biological activity of GDF15.

30. The method of claim 29, wherein the GDF15 active domain comprises an amino acid sequence selected from one of the following group, or having at least 85%, 90%, 95% or 99% sequence identity to one of the following group:
SEQ ID NO: 46;
1-14 amino acid truncations at the N-terminal of SEQ ID NO:46, and/or 1-3 amino acid substitutions in SEQ ID NO:46.

31. The method of claim 30, wherein the GDF15 active domain comprises an amino acid sequence selected from one of the following group:
SEQ ID NO: 46;
1-14 amino acid truncations at the N-terminal of SEQ ID NO:46, and/or 1-3 amino acid substitutions in SEQ ID NO:46.

32. The method of claim 30, wherein the position of amino acid substitution in SEQ ID NO:46 is selected from one, two or three of the following group: position 5, position 6, position 21, position 26, position 30, position 47, position 54, position 55, position 57, position 67, position 69, position 81, position 94, position 107;
preferably, the amino acid substitution in the SEQ ID NO: 46 is selected from one, any two of the different positions, or any three of the different positions: D5E, H6D, H6E, R21Q, R21H, D26E, A30S, A47D, A54S, A55E, M57T, R67Q, K69R, A81S, T94E, K107Q.

33. The method of claim 30, wherein the N-terminal of SEQ ID NO: 46 is truncated by 3, 4 or 14 amino acids.

34. The method of any one of claims 30 to 33, wherein the GDF15 active domain comprises an amino acid sequence of one of the following group, or an amino acid sequence having at least 85%, 90%, 95% or 99% sequence identity to one of the following group: SEQ ID NO: 46-66.

35. The method of claim 34, wherein the GDF15 active domain comprises an amino acid sequence of one of the following group, or an amino acid sequence having at least 95% sequence identity to one of the following group: SEQ ID NO: 46-66;
preferably, the GDF15 active domain comprises an amino acid sequence of one of the following group: SEQ ID NO: 46-66.

36. The method of any one of claims 21 to 35, wherein the fusion protein comprises an amino acid sequence of one of the following group, or an amino acid sequence having at least 85%, 90%, 95% or 99% sequence identity to one of the following group: SEQ ID NO: 67-78, SEQ ID NO: 80-102, SEQ ID NO: 105-118.

37. The method of claims 36, wherein the fusion protein comprises an amino acid sequence of one of the following group, or an amino acid sequence having at least 95% sequence identity to one of the following group: SEQ ID NO: 67-78, SEQ ID NO: 80-102, SEQ ID NO: 105-118;
preferably, the fusion protein comprises an amino acid sequence of one of the following group: SEQ ID NO: 67-78, SEQ ID NO: 80-102, SEQ ID NO: 105-118.

38. A method of treating obesity-related diseases using a homodimeric fusion protein, wherein the homodimeric fusion protein comprises the fusion protein of any one of claims 21 to 27.

39. A method of treating obesity-related diseases using a biological material, wherein the biological material is any one of the following (i), (ii), (iii):

(i) a nucleic acid comprising a nucleotide sequence encoding the fusion protein of any one of claims 1 to 17;
(ii) a vector comprising the nucleic acid of (i);
(iii) a host cell containing the nucleic acid of (i) and/or the vector of (ii).

40. A method of treating obesity-related diseases using a pharmaceutical composition, wherein the pharmaceutical composition comprises the homodimeric fusion protein of claim 38 as an active ingredient, and the homodimeric fusion protein is present in the pharmaceutical composition in a therapeutically effective amount; preferably, the pharmaceutical composition further comprises a pharmaceutically acceptable carrier.

Figure 1

Figure 2

Figure 3

Figure 4

Figure 5

Figure 6

Figure 7

Figure 8

Figure 9

Figure 10

Figure 11

Figure 12a

Figure 12b

Figure 13

Figure 14

Figure 15

Figure 16a

Figure 16b

Figure 17

Figure 18

Figure 19

Figure 20

Figure 21

## INTERNATIONAL SEARCH REPORT

| International application No. |
| --- |
| **PCT/CN2022/114143** |

**A. CLASSIFICATION OF SUBJECT MATTER**

C07K 14/475(2006.01)i;  A61K 38/18(2006.01)i;  A61K 47/68(2017.01)i;  A61P 3/04(2006.01)i

According to International Patent Classification (IPC) or to both national classification and IPC

**B. FIELDS SEARCHED**

Minimum documentation searched (classification system followed by classification symbols)

C07K; A61K; A61P

Documentation searched other than minimum documentation to the extent that such documents are included in the fields searched

Electronic data base consulted during the international search (name of data base and, where practicable, search terms used)

CNABS, VEN, CNKI, CNTXT, USTXT, EPTXT, PUBMED, ISI WEB, NCBI, EMBL-EBI, STNext: 申请人/发明人, 融合蛋白, 生长分化因子15, 巨噬细胞抑制细胞因子-1, Fc, 突变, GDF15, Fc-GDF15, GDF15, Fc, E356, K439, mutant肥胖, obesity, SEQ ID NOs: 1-118

**C. DOCUMENTS CONSIDERED TO BE RELEVANT**

| Category* | Citation of document, with indication, where appropriate, of the relevant passages | Relevant to claim No. |
| --- | --- | --- |
| X | WO 2021/107603 A2 (YUHAN CORP.) 03 June 2021 (2021-06-03) claims 1-25 | 1, 21 |
| Y | WO 2021/107603 A2 (YUHAN CORP.) 03 June 2021 (2021-06-03) claims 1-25 | 2-20, 22-40 |
| Y | CN 113286812 A (XILIO DEVELOPMENT INC.) 20 August 2021 (2021-08-20) description, paragraph [0427] | 2-20, 22-40 |
| X | WO 2020/218827 A1 (LG CHEMICAL LTD.) 29 October 2020 (2020-10-29) claims 1-21 | 1, 21 |
| Y | WO 2020/218827 A1 (LG CHEMICAL LTD.) 29 October 2020 (2020-10-29) claims 1-21 | 2-20, 22-40 |
| X | US 2021/0147500 A1 (AMGEN INC.) 20 May 2021 (2021-05-20) abstract, and description, paragraphs [0021]-[0106] | 1, 21 |
| Y | US 2021/0147500 A1 (AMGEN INC.) 20 May 2021 (2021-05-20) abstract, and description, paragraphs [0021]-[0106] | 2-20, 22-40 |

☑ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **17 October 2022** | **23 November 2022** |

| Name and mailing address of the ISA/CN | Authorized officer |
| --- | --- |
| **China National Intellectual Property Administration (ISA/ CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

| International application No. |
| --- |
| **PCT/CN2022/114143** |

| C. | DOCUMENTS CONSIDERED TO BE RELEVANT | |
| --- | --- | --- |
| Category* | Citation of document, with indication, where appropriate, of the relevant passages | Relevant to claim No. |
| Y | WO 2021/076564 A1 (DRAGONFLY THERAPEUTICS INC.) 22 April 2021 (2021-04-22) description, paragraph [0180] | 2-20, 22-40 |
| X | FUNG, E. et al. "Fc-GDF15 glyco-engineering and receptor binding affinity optimization for body weight regulation." *Scientific Reports.*, Vol. 11, 26 April 2021 (2021-04-26), article number 8921, pp. 1-12 | 1, 21 |
| Y | FUNG, E. et al. "Fc-GDF15 glyco-engineering and receptor binding affinity optimization for body weight regulation." *Scientific Reports.*, Vol. 11, 26 April 2021 (2021-04-26), article number 8921, pp. 1-12 | 2-20, 22-40 |

Form PCT/ISA/210 (second sheet) (January 2015)

**INTERNATIONAL SEARCH REPORT**

| International application No. |
|---|
| **PCT/CN2022/114143** |

| **Box No. I** | **Nucleotide and/or amino acid sequence(s) (Continuation of item 1.c of the first sheet)** |
|---|---|

1. With regard to any nucleotide and/or amino acid sequence disclosed in the international application, the international search was carried out on the basis of a sequence listing:

    a. ☑ forming part of the international application as filed:

        ☑ in the form of an Annex C/ST.25 text file.

        ☐ on paper or in the form of an image file.

    b. ☐ furnished together with the international application under PCT Rule 13*ter*.1(a) for the purposes of international search only in the form of an Annex C/ST.25 text file.

    c. ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

    [1] The actually submitted sequence table is an XML file in Standard ST.26.

Form PCT/ISA/210 (continuation of first sheet) (January 2015)

**INTERNATIONAL SEARCH REPORT**

International application No.

**PCT/CN2022/114143**

| Box No. II | Observations where certain claims were found unsearchable (Continuation of item 2 of first sheet) |
|---|---|

This international search report has not been established in respect of certain claims under Article 17(2)(a) for the following reasons:

1. ☑ Claims Nos.: **21-40**
   because they relate to subject matter not required to be searched by this Authority, namely:

   [1]   Claims 21-40 relate to a method for preventing and/or treating diseases, belonging to the methods for treating diseases as defined in PCT Rule 39.1(iv). The present report was formed on the basis of a corresponding pharmaceutical use.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (January 2015)

INTERNATIONAL SEARCH REPORT
Information on patent family members

International application No.

**PCT/CN2022/114143**

| Patent document cited in search report | | | Publication date (day/month/year) | Patent family member(s) | | | Publication date (day/month/year) |
|---|---|---|---|---|---|---|---|
| WO | 2021/107603 | A2 | 03 June 2021 | EP | 4065597 | A2 | 05 October 2022 |
| | | | | KR | 20210065057 | A | 03 June 2021 |
| | | | | AU | 2020394255 | A1 | 09 June 2022 |
| | | | | CA | 3161302 | A1 | 03 June 2021 |
| CN | 113286812 | A | 20 August 2021 | EP | 3856764 | A1 | 04 August 2021 |
| | | | | US | 2022306716 | A1 | 29 September 2022 |
| | | | | JP | 2022502088 | A | 11 January 2022 |
| | | | | US | 2021002343 | A1 | 07 January 2021 |
| | | | | PH | 12021550648 | A1 | 14 February 2022 |
| | | | | AU | 2019347751 | A1 | 29 April 2021 |
| | | | | IL | 281776 | A | 31 May 2021 |
| | | | | BR | 112021005907 | A2 | 10 August 2021 |
| | | | | SG | 11202102777 P | A | 29 April 2021 |
| | | | | WO | 2020069398 | A1 | 02 April 2020 |
| | | | | KR | 20210087027 | A | 09 July 2021 |
| | | | | US | 2022002370 | A1 | 06 January 2022 |
| | | | | CA | 3112989 | A1 | 02 April 2020 |
| WO | 2020/218827 | A1 | 29 October 2020 | JP | 2022530216 | A | 28 June 2022 |
| | | | | KR | 20200124176 | A | 02 November 2020 |
| | | | | AU | 2020260931 | A1 | 09 December 2021 |
| | | | | PE | 20220500 | A1 | 07 April 2022 |
| | | | | CL | 2021002757 | A1 | 10 June 2022 |
| | | | | CN | 113767112 | A | 07 December 2021 |
| | | | | CO | 2021014413 | A2 | 19 November 2021 |
| | | | | KR | 20210080339 | A | 30 June 2021 |
| | | | | EP | 3978518 | A1 | 06 April 2022 |
| | | | | TW | 202100563 | A | 01 January 2021 |
| | | | | IL | 287343 | A | 01 December 2021 |
| | | | | SG | 11202111570 T | A | 29 November 2021 |
| | | | | CA | 3136969 | A1 | 29 October 2020 |
| US | 2021/0147500 | A1 | 20 May 2021 | KR | 20200140878 | A | 16 December 2020 |
| | | | | JP | 2020533271 | A | 19 November 2020 |
| | | | | BR | 112020020823 | A2 | 19 January 2021 |
| | | | | EA | 202092419 | A1 | 05 February 2021 |
| | | | | JO | P20200258 | A1 | 09 October 2019 |
| | | | | AR | 114476 | A1 | 09 September 2020 |
| | | | | CO | 2020012563 | A2 | 30 October 2020 |
| | | | | TW | 202003586 | A | 16 January 2020 |
| | | | | US | 2022017584 | A1 | 20 January 2022 |
| | | | | MA | 52205 | A | 17 February 2021 |
| | | | | PH | 12020551655 | A1 | 26 July 2021 |
| | | | | CL | 2020002585 | A1 | 04 December 2020 |
| | | | | IL | 277842 | A | 30 November 2020 |
| | | | | CA | 3096097 | A1 | 17 October 2019 |
| | | | | PE | 20201350 | A1 | 30 November 2020 |
| | | | | UY | 38177 | A | 31 July 2019 |
| | | | | AU | 2019253462 | A1 | 26 November 2020 |
| | | | | EP | 3773656 | A1 | 17 February 2021 |
| | | | | SG | 11202009884 Y | A | 27 November 2020 |
| | | | | CN | 111954537 | A | 17 November 2020 |

Form PCT/ISA/210 (patent family annex) (January 2015)

**INTERNATIONAL SEARCH REPORT**
Information on patent family members

International application No.

**PCT/CN2022/114143**

| Patent document cited in search report | | | Publication date (day/month/year) | Patent family member(s) | | | Publication date (day/month/year) |
|---|---|---|---|---|---|---|---|
| | | | | CR | 20200510 | A | 26 November 2020 |
| | | | | WO | 2019199685 | A1 | 17 October 2019 |
| | | | | JP | 2022095856 | A | 28 June 2022 |
| WO | 2021/076564 | A1 | 22 April 2021 | AR | 120223 | A1 | 02 February 2022 |
| | | | | TW | 202128759 | A | 01 August 2021 |
| | | | | BR | 112022007128 | A2 | 05 July 2022 |
| | | | | KR | 20220083770 | A | 20 June 2022 |
| | | | | CA | 3153858 | A1 | 22 April 2021 |
| | | | | IL | 292261 | A | 01 June 2022 |
| | | | | EP | 4045538 | A1 | 24 August 2022 |
| | | | | CO | 2022004757 | A2 | 30 September 2022 |
| | | | | AU | 2020368163 | A1 | 28 April 2022 |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202110977378

- WO 2019195091 A

**Non-patent literature cited in the description**

- **JOHNEN H et al.** *Nat Med,* 2007
- **XIONG Y et al.** *Sci Transl Med,* 2017

- **EDELMAN et al.** The covalent structure of an entire γG immunoglobulin molecule. *Proc. Natl. Acad. Sci., USA,* 1969